# EUROPEAN PATENT APPLICATION

(11) **EP 4 620 449 A2**
(43) Date of publication of application: **24.09.2025**
(21) Application number: 25151710.8
(22) Date of filing: 14.01.2025
(51) Int. Cl.: A61H 23/02, A61N 1/00

(54) **SKIN CARE DEVICE**

(30) Priority: 18.03.2024 KR 20240036952
(71) Applicant: ACCESS BUSINESS GROUP INTERNATIONAL LLC, Ada, Michigan 49355 (US)
(72) Inventor: JO, Hae, Hanam-si (KR); JEON, Soha, Seoul (KR); CHANG, Kiyoung, Seoul (KR)
(74) Representative: Haseltine Lake Kempner LLP

(57) **Abstract**

A device to be used for skin care is provided. The device includes a housing, a head mount, a first head, a second head and a controller. The head mount is coupled to the housing. The first head includes a first contact portion making contact with a target object. The first head is detachably mounted to the head mount along a first central axis of the head mount and is electrically connected to the head mount. The second head includes a second contact portion formed to surround the first contact portion and making contact with the target object together with the first contact portion. The second head is detachably mounted to the first head along the first central axis and is electrically connected to the first head. The controller controls the first head and the second head.

## Description

### TECHNICAL FIELD

The present disclosure relates to a technology that is used for skin care.

### BACKGROUND

A skin care device that can be used for skin care is already known. For example, a user can use the skin care device by holding it in his or her hand and bringing said device into contact with his or her facial skin.

Such skin care device can provide particular actions to the skin. Further, the skin care device can be used together with skin care products applied to the user's skin. The skin care device can perform the action of assisting the absorption of the skin care products into the user's skin.

### SUMMARY

At least one embodiment of the present disclosure provides a technology that can perform one or more actions for skin care in one device.

At least one embodiment of the present disclosure provides a technology that can easily combine and replace heads for performing one or more actions.

At least one embodiment of the present disclosure provides enhanced usability, enhanced manipulability and differentiated usage experiences to a user.

Embodiments disclosed herein relate to a device that can be used for skin care. The device according to one embodiment includes a housing, a head mount, a first head, a second head and a controller. The head mount is configured to be coupled to the housing. The first head includes a first contact portion making contact with a target object. The first head is configured to be detachably mounted to the head mount along a first central axis of the head mount and to be electrically connected to the head mount. The second head includes a second contact portion formed to surround the first contact portion and making contact with the target object together with the first contact portion. The second head is configured to be detachably mounted to the first head along the first central axis and to be electrically connected to the first head. The controller is configured to control the first head and the second head.

In one embodiment, the head mount is configured to rotate about the first central axis to be coupled to an end portion of the housing in a first direction or a second direction. The controller is configured to supply electrical power to the first head and the second head upon detecting that the head mount is coupled to the end portion of the housing in the first direction, and to further cut off the electrical power upon detecting that coupling of the head mount to the end portion of the housing in the first direction is released.

In one embodiment, the housing includes a detection switch, which is disposed inside the housing and transmits a detection signal to the controller upon coupling of the head mount to the end portion of the housing in the first direction. The head mount includes a switch operating portion that may turn on or off the detection switch by rotation of the head mount.

In one embodiment, a mounting plate is formed at the end portion of the housing to which the head mount is coupled, and the mounting plate is inclined at a predetermined tilting angle with respect to a second central axis of the housing. The head mount is configured to rotate about the first central axis to be coupled to the mounting plate in the first direction or the second direction. When the head mount is coupled in the first direction, the first central axis of the head mount is tilted at the tilting angle with respect to the second central axis of the housing. When the head mount is coupled in the second direction, the first central axis of the head mount is parallel or coaxial with the second central axis of the housing.

In one embodiment, the head mount includes a ball that is elastically pressed toward the mounting plate. The mounting plate includes a detent to which the ball is releasably inserted when the head mount is coupled to the end portion of the housing in the first direction or the second direction.

In one embodiment, the head mount includes a locking latch that is elastically pressed toward the first central axis, and the locking latch includes a push button disposed in an outer peripheral surface of the head mount. The first head includes a locking groove to which the locking latch is releasably coupled.

In one embodiment, the first head includes a disk, a shaft and a protruding end. The disk is detachably mounted to the head mount. The shaft protrudes from the disk along the first central axis. The protruding end is formed in the shaft by the first contact portion. The second head includes a first through hole and a first annular body. The shaft of the first head is inserted to the first through hole along the first central axis. The first annular body includes an upper end formed along a circumference of the first through hole by the second contact portion, and is further disposed on the disk of the first head.

In one embodiment, when the second head is mounted to the first head, the first contact portion of the protruding end and the second contact portion of the upper end are positioned concentrically with respect to the first central axis.

In one embodiment, the first head includes a first magnet disposed inside the disk, while the second head includes a second magnet disposed inside the first annular body and corresponding to the first magnet.

In one embodiment, the shaft of the first head includes a key formed along the first central axis in an outer peripheral surface of the shaft, and the first through hole of the second head includes a key groove into which the key is inserted.

The device according to one embodiment further includes a third head, which is configured to be detachably mounted to the first head along the first central axis and to be electrically connected to the first head. The third head includes a third contact portion formed to surround the first contact portion and making contact with the target object together with the first contact portion. The second head and the third head are detachably and selectively mounted to the first head. The controller is configured to control the third head.

In one embodiment, the third head includes a second through hole and a second annular body. The shaft of the first head is inserted to the second through hole along the first central axis. The second annular body includes an upper end formed along a circumference of the second through hole by the third contact portion, and is further disposed on the disk of the first head.

In one embodiment, the third head includes a third magnet disposed inside the second annular body and corresponding to the first magnet.

In one embodiment, the third through hole of the third head includes a key groove into which the key of the first head is inserted.

In one embodiment, the first contact portion includes an ultrasonic element for applying an ultrasonic wave for sonophoresis to the target object. One of the second contact portion and the third contact portion includes a first electrode, while the other of the second contact portion and the third contact portion includes a second electrode and a third electrode. The first electrode is configured to alternately apply an electric wave to the target object. The second electrode is configured to apply a first electrical current for electroporation to the target object. The third electrode is configured to apply a second electrical current for iontophoresis to the target object. The housing includes a counter electrode, which is disposed in an outer peripheral surface of the housing and is configured to apply the second electrical current to the target object.

In one embodiment, the first electrode may include a pair of electrodes formed in a circular arc shape so as to surround a portion of the ultrasonic element in a circumferential direction of the first central axis, respectively.

In one embodiment, the second electrode may include a positive electrode and a negative electrode for applying the first electrical current to the target object. Each of the positive electrode of the second electrode, the negative electrode of the second electrode and the third electrode may be formed in a circular arc shape so as to surround a portion of the ultrasonic element in a circumferential direction of the first central axis.

The device according to one embodiment includes an electrical power source disposed inside the housing. The electrical power source is configured to be controlled by the controller to supply electrical power to the first head, the second head and the third head.

The device according to one embodiment includes a display disposed on the housing. The display is configured to display information directed to the operating state of the device and may be controlled by the controller.

The device according to one embodiment includes a speaker disposed inside the housing. The speaker is configured to emit information directed to the operating state of the device by sound and may be controlled by the controller.

The device according to one embodiment includes a memory card reader disposed in the housing. The memory card reader is configured to accommodate a memory card and is connected to the controller.

The device according to one embodiment includes a cradle configured to support the housing, the head mount and the first head that are sequentially coupled along the second central axis of the housing. The cradle includes a first docking portion supporting the lower end portion of the housing as well as a second docking portion spaced apart from the first docking portion along the second central axis and supporting the head mount and the first head.

In one embodiment, the first docking portion may include a docking hole receiving the lower end portion of the housing along the second central axis. The second docking portion may include a docking surface formed complementarily to an outer peripheral surface of the head mount and an outer peripheral surface of the first head, as well as a guide groove formed in the docking surface and guiding the head mount and the first head to the docking surface.

In one embodiment, the housing may include a female charging terminal disposed in the lower end portion of the housing and charging the electrical power source. The first docking portion may include a male charging terminal electrically connected to the female charging terminal and configured to be pressed by the female charging terminal.

In one embodiment, the cradle may include an ultraviolet light emitter disposed so as to emit ultraviolet light toward the first head and the second head.

In one embodiment, the cradle may include a sterilization indicator providing information directed to the operation of the ultraviolet light emitter.

In one embodiment, the cradle may include a head cover configured to be rotatable so as to cover the ultraviolet light emitter and the first and second heads docked to the second docking portion, and a cover switch detecting rotation of the head cover to operate the ultraviolet light emitter and the sterilization indicator.

In one embodiment, the cradle may include a head keeping recess located between the first docking portion and the second docking portion and formed so as to accommodate the second head or the third head.

According to at least one embodiment of the present disclosure, a technology that can perform one or more actions for skin care in one device can be provided.

According to at least one embodiment of the present disclosure, a technology that can easily combine and replace the heads for performing one or more actions can be provided.

According to at least one embodiment of the present disclosure, enhanced usability, enhanced manipulability and differentiated usage experiences can be provided to a user.

### BRIEF DESCRIPTION OF DRAWINGS

FIG. 1 is a perspective view showing a device according to one embodiment.
FIG. 2 is an exploded perspective view of the device shown in FIG. 1.
FIG. 3 is an exploded perspective view of a housing shown in FIG. 1.
FIG. 4 is a sectional view showing a head mount and an upper portion of the housing and shows that the head mount rotates to be coupled to the housing in a first direction and a second direction.
FIG. 5 is a perspective view showing a lower portion of the head mount and the upper portion of the housing.
FIG. 6 is a perspective view showing that the head mount is coupled to the housing in the first direction shown in FIG. 4.
FIG. 7 is a perspective view showing the lower portion of the head mount and an upper end portion of the housing.
FIG. 8 is a sectional perspective view showing the housing, the head mount, and a first head.
FIG. 9 is a perspective view showing the head mount, the first head, and a second head of the device according to one embodiment.
FIG. 10 is an exploded perspective view of the first head shown in FIG. 9.
FIG. 11 is an exploded perspective view of the second head shown in FIG. 9.
FIG. 12 is a sectional view of the head mount, the first head, and the second head shown in FIG. 9.
FIG. 13 is a perspective view showing a device according to one embodiment, and shows a third head mounted to the first head.
FIG. 14 is a perspective view showing the head mount, the first head and the third head shown in FIG. 13.
FIG. 15 is an exploded perspective view of the third head shown in FIG. 14.
FIG. 16 is a sectional view of the head mount, the first head, and the third head shown in FIG. 14.
FIG. 17 is a block diagram schematically showing components of a device according to one embodiment.
FIG. 18 is a perspective view showing a device according to one embodiment including a cradle.
FIG. 19 is a partial perspective view of the cradle, showing a first docking portion of the cradle shown in FIG. 18.
FIG. 20 is a partial perspective view of the housing, showing the lower end portion of the housing shown in FIG. 18.
FIG. 21 is a partial perspective view of the cradle, showing a second docking portion of the cradle shown in FIG. 18.
FIG. 22 is a partial perspective view showing the housing, the head mount, the first head and the second head shown in FIG. 18.
FIG. 23 is a perspective view showing the second docking portion and a head cover of the cradle shown in FIG. 18.
FIG. 24 is a sectional view showing a longitudinal sectional shape of the cradle shown in FIG. 18.

### DETAILED DESCRIPTION

Embodiments of the present disclosure are illustrated for the purpose of explaining the technical idea of the present disclosure. The scope of the rights according to the present disclosure is not limited to the embodiments presented below or the detailed descriptions of such embodiments.

All technical terms and scientific terms used in the present disclosure include meanings that are commonly understood by those of ordinary skill in the technical field to which the present disclosure pertains unless otherwise defined. All terms used in the present disclosure are selected for the purpose of describing the present disclosure more clearly, and are not selected to limit the scope of the rights according to the present disclosure.

Expressions such as "comprising," "including," "having" and the like used in the present disclosure are to be understood as open-ended terms having the possibility of encompassing other embodiments, unless otherwise mentioned in the phrase or sentence containing such expressions.

Singular expressions described in the present disclosure may encompass plural expressions unless otherwise stated, which will also apply to singular expressions recited in the claims.

Expressions such as "first," "second," etc. used in the present disclosure are used to distinguish a plurality of elements from one another, and are not intended to limit an order or importance of the elements.

In the present disclosure, the description that one element is "connected" or "coupled" to another element should be understood to indicate that the aforesaid one element may be directly connected or coupled to the aforesaid another element, and should be further understood that the aforesaid one element may be connected or coupled to the aforesaid another element via a new element.

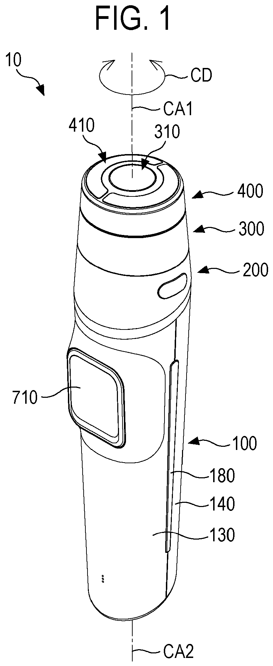

Directional terms "upward" and "downward" used in the present disclosure are based on the orientation of a device shown in FIG. 1.

Hereinafter, the embodiments of the present disclosure are described with reference to the accompanying drawings. Like reference numerals in the accompanying drawings denote like or corresponding elements. Further, in the following description of the embodiments, redundant descriptions for the same or corresponding elements may be omitted. However, even if the descriptions of the elements are omitted, such elements are not intended to be excluded in any embodiment.

The embodiments disclosed below and the embodiments shown in the attached drawings relate to a device that can be used for skin care. The device according to the embodiments can be formed so as to be held by a user's hand, and a user can use the device for purposes of skin care in a state where a contact portion of the device is in contact with the user's skin. By way of example, the device according to the embodiments can be used to regenerate aged collagen and elastin within a skin tissue, or to regenerate skin cells, or to effectively deliver skin care products into the skin. The user can use the device according to the embodiments for purposes of skin care, but a target object to which the device according to the embodiments can be applied is not limited to the skin.

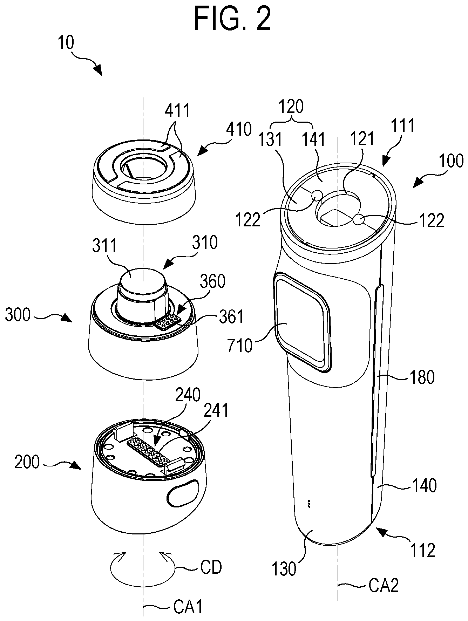

FIG. 1 is a perspective view showing a device according to one embodiment, while FIG. 2 is an exploded perspective view of the device shown in FIG. 1. Reference is made to FIG. 1 and FIG. 2 in the below descriptions.

The device 10 according to one embodiment includes a housing 100, a head mount 200 configured to be coupled to the housing, a first head 300 configured to detachably mounted to the head mount, and a second head 400 configured to be detachably mounted to the first head. Further, the device 10 includes a controller configured to control the first head 300, the second head 400, and the components of the device that can be installed in the housing 100. The first head 300 and the second head 400 may make contact with a target object (e.g., a user's skin), and may perform actions for skin care. The housing 100 and the head mount 200, which are coupled to each other, may constitute a singe module. The first head 300 mounted to the head mount 200 may constitute a single module, while the second head 400 mounted to the first head may constitute a single module.

The housing 100 is a part which the user can hold when he or she uses the device 10. The housing 100 is an external member of the device 10, and accommodates, within its internal space, components functioning for the operation of the device. According to one embodiment of the device, the housing 100 includes a front housing 130 and a rear housing 140. The front housing and the rear housing are coupled to each other, thereby forming the internal space of the housing 100. The housing 100 is formed in an approximately cylindrical shape and thus defines two end portions in a longitudinal direction. The head mount 200 is coupled to one end portion 111 of the housing in the longitudinal direction. In the below descriptions, the end portion 111, to which the head mount is coupled, is referred to as an upper end portion, while an end portion, which is located opposite to the upper end portion 111 in the longitudinal direction, is referred to as a lower end portion 112.

The housing 100 includes a mounting plate 120 formed in the upper end portion 111. The head mount 200 is rotatably coupled to the mounting plate 120 of the housing. An upper end portion of the front housing 130 and an upper end portion of the rear housing 140 are coupled to each other and can form the mounting plate 120 of the housing 100.

The first head 300 is mounted to the head mount 200. The head mount 200 supports the first head 300 and can connect the first head 300 to the housing 100. The head mount 200 may be formed in a cylindrical shape and has a first central axis CA1. The first central axis CA1 may mean an imaginary axis extending through a center of a cross-sectional shape of the head mount 200. The head mount 200 may be coupled to the upper end portion 111 of the housing. According to one embodiment of the device, the head mount 200 may be coupled to the upper end portion 111 of the housing so as to rotate about the first central axis CA1. Further, according to one embodiment of the device, the head mount 200 may be coupled to the upper end portion 111 of the housing such that the first central axis CA1 is parallel with a second central axis CA2 of the housing 100 or the first central axis is tilted with respect to the second central axis. The second central axis CA2 of the housing 100 may mean an imaginary axis extending through a center of the mounting plate 120 in the longitudinal direction of the housing.

Electrical power for operation of the first head 300 may be supplied to the first head through the head mount 200. For electrical connection of the head mount 200 and the first head 300, the head mount 200 may include a first connector 240, and the first head 300 may include a second connector. The first connector 240 may be disposed in an upper surface of the head mount 200, and may be a male connector. The second connector may be disposed in a lower surface of the first head 300 so as to correspond to the first connector 240, and may be a female connector.

By way of example, the first connector 240 may be a pogo pin connector, while the second connector of the first head 300 may be a connector that has terminals capable of making contact with the respective pogo pins of the pogo pin connector. Each pogo pin 241 of the pogo pin connector may include a cylindrical barrel, a spring inserted to the barrel, and a plunger coupled to one end portion of the barrel so as to move along the barrel and pressed by the spring. Thus, when the first connector 240 and the second connector are coupled, the plunger of the pogo pin can be elastically pressed toward an inside of the barrel. Since the first connector 240 may be configured as a pogo pin connector, coupling and decoupling of the head mount 200 and the first head 300 can be easily performed. When the first head 300 is detachably mounted to the head mount 200, the first connector 240 and the second connector are electrically connected to each other. Thus, the first head 300 can be electrically connected to the head mount 200, and electrical power can be supplied to the first head 300 through the head mount 200.

The first head 300 is detachably mounted to the head mount 200 along the first central axis CA1. When the device is not used, the first head 300 may be detached from the head mount 200. The first head 300 includes a first contact portion 310, which makes contact with a target object (e.g., a user's skin) when the device is used. The first head 300 may include an electronic element that can perform actions for skin care, and the first contact portion 310 may include a surface of such an electronic element. According to one embodiment of the device, the first contact portion 310 may be positioned at an upper end of the first contact portion 310, and may include an ultrasonic element 311. An ultrasonic wave applying surface of the ultrasonic element 311 may form the first contact portion 310.

The second head 400 is detachably mounted to the first head 300 along the first central axis CA1. When the device is not used, the second head 400 may be detached from the first head 300. The second head 400 includes a second contact portion 410, which makes contact with the target object when the device is used. The second head 400 is formed in an annular shape so as to surround the first contact portion 310 of the first head in a circumferential direction CD of the first central axis CA1. Further, the second contact portion 410 is formed so as to make contact with the target object together with the first contact portion 310 when the device is used. Specifically, in a state where the second head 400 is mounted to the first head 300, the first contact portion 310 of the first head and the second contact portion 410 of the second head are positioned concentrically with respect to the first central axis CA1, and can make contact with the target object together. Thus, when the device is used, the first contact portion 310 and the second contact portion 410, which are positioned concentrically, may make contact with the target object together, and the skin care actions included in the first head and the second head can be simultaneously performed to the target object.

The second head 400 may include an electrode for performing actions for skin care, and the second contact portion 410 may include a surface of such an electrode. According to one embodiment of the device, the second contact portion 410 may be positioned at an upper end of the second head 400, and may include a first electrode 411 configured to apply an electric wave to the target object.

Electrical power for operation of the second head 400 may be supplied to the second head through the first head 300. For electrical connection of the first head 300 and the second head 400, the first head 300 may include a third connector 360, and the second head 400 may include a fourth connector. The third connector 360 is electrically connected to the second connector of the first head. The third connector 360 may be spaced apart from the first contact portion 310 and be disposed in a surface of the first head 300. The fourth connector may be disposed in a lower surface of the second head 400 so as to correspond to the third connector 360. The third connector 360 may be the above-described pogo pin connector, and may include the same pogo pin 361 as the pogo pin 241. The fourth connector may be a connector that has terminals capable of making contact with the respective pogo pins 361 of the third connector. Since the third connector 360 may be configured as a pogo pin connector, coupling and decoupling of the first head 300 and the second head 400 can be easily performed. When the second head 400 is detachably mounted to the first head 300, the third connector 360 and the fourth connector are electrically connected to each other, and the second head 400 is electrically connected to the first head 300. The electrical power can be supplied to the second head 400 through the head mount 200 and the first head 300.

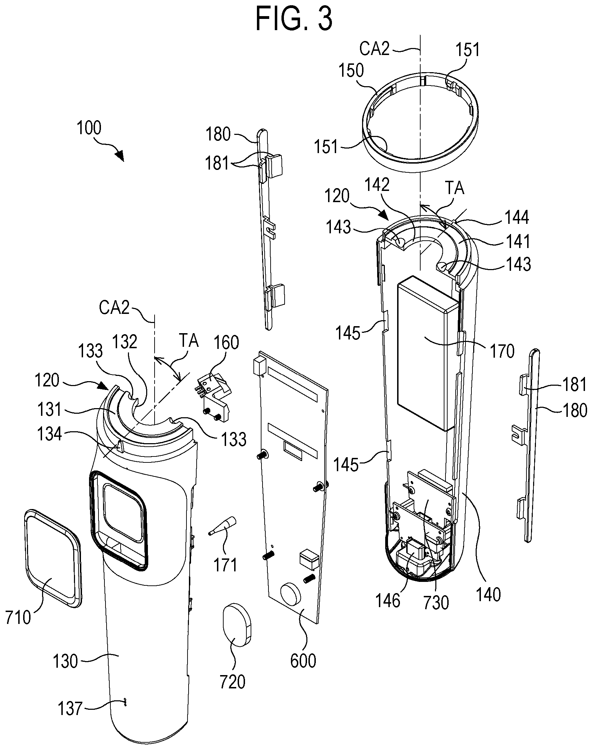

FIG. 3 is an exploded perspective view of the housing shown in FIG. 1. Reference is made to FIG. 3 in the below description.

The device includes an electrical power source configured to supply electrical power for operation of the device. The electrical power source may be positioned outside the device 10, and may be connected to the device through an adaptor. Alternatively, the electrical power source may be disposed inside the housing 100. The device according to one embodiment includes an electrical power source 170 disposed inside the housing 100. The electrical power source 170 may be a rechargeable battery. Alternatively, the electrical power source 170 may be a replaceable battery. The device, which includes the electrical power source 170 inside the housing 100, is portable. The electrical power source 170 is configured to supply electrical power to the first head and the second head. Further, the electrical power source 170 is configured to supply electrical power to electrical components disposed in the housing 100. The electrical power source 170 is electrically connected to the controller and may be controlled by the controller.

A charging port 146 for charging the electrical power source 170 may be installed at the lower end portion of the housing 100. For example, the charging port 146 may have a USB-C type terminal. The charging port 146 may be fixed to the rear housing 140 so as to communicate with a lower end portion of the rear housing 140. Further, the device 10 may include a charging indicator 171 that indicates a charged state of the electrical power source 170. The charging indicator 171 is fixed to an inner peripheral surface of the front housing 130, and may be controlled by the controller. By way of example, the charging indicator 171 may include a light emitting diode capable of indicating the charged state.

The device includes the controller 600 that controls operations of the electrical components of the device. By way of example, the controller 600 may include a board, a processing processor mounted on the board, and a plurality of circuits formed on the board, connected to the processing processor, and configured to perform respective functions of the device. The controller 600 may be disposed inside the housing 100. Alternatively, a portion of the controller 600 may be disposed inside the first head and inside the second head. The controller 600 is electrically connected to the electrical power source 170. The controller 600 is electrically connected to the first connector of the head mount.

The housing 100 includes a counter electrode 180 disposed in an outer peripheral surface of the housing. In the device according to one embodiment, a pair of counter electrodes 180 are disposed between the front housing 130 and the rear housing 140, and are exposed to an outside of the housing 100. The counter electrodes 180 are made of a metallic material, and may function as an electrode for applying an electrical current for iontophoresis to the target object. The counter electrodes 180 may be electrically connected to the controller 600. When a user holds the housing 100, the counter electrodes 180 can make contact with the user's hand. Each of the counter electrodes 180 has an engagement portion consisting of a pair of engaging hooks 181. The rear housing 140 has, in its inner peripheral surface, an engaging groove 145 with which one of the pair of engaging hooks 181 is engaged. The front housing 130 has, in its inner peripheral surface, an engaging groove with which the other of the pair of engaging hooks 181 is engaged, and which corresponds to the engaging groove 145. The pair of engaging hooks 181 are coupled to the engaging grooves of the front and rear housings 130 and 140, respectively, thereby situating the counter electrodes 180 between the front housing 130 and the rear housing 140.

The device according to one embodiment includes a display 710 disposed on the housing 100. By way of example, the display 710 may be an LCD, and may be fixed to the front housing 130 so as to be exposed to the outside of the front housing 130. The display 710 may be a touch screen display that can receive an input from a user. The display 710 is controlled by the controller 600, and is configured to display information directed to an operating state of the device.

The device according to one embodiment includes a speaker 720, which is disposed inside the housing 100 and is controlled by the controller 600. The speaker 720 may be mounted on the controller 600, and a sound hole 137 may be formed in the front housing 130 so as to correspond to the speaker. The speaker 720 is configured to emit information directed to the operating state of the device by sound such as a voice. Accordingly, when the user attempts to use the device or while the user is using the device, the user can receive the information directed to the operation of the device by sound guidance.

The device according one embodiment includes a memory card reader 730, which is disposed inside the housing 100 and is connected to the controller 600. The memory card reader 730 is configured to accommodate a memory card (e.g., an SD card). The memory card reader 730 may be fixed to the rear housing 140 so as to communicate with a lower end portion of the rear housing 140. The rear housing 140 may have a card hole, through which the memory card can be inserted to the memory card reader 730, and a cover covering the card hole. The memory card may store information directed to the operations of the first head and the second head. Further, the memory card may store information directed to the operation of an additional head which may be mounted to the first head.

The mounting plate 120, to which the head mount is rotatably mounted, may be disposed so as to be orthogonal to the second central axis CA2 of the housing. Alternatively, the mounting plate 120 may be disposed so as to be inclined with respect to the second central axis CA2. In the device according to one embodiment, the upper end portion of the front housing 130 and the upper end portion of the rear housing 140 are coupled to each other to form the mounting plate 120, and the mounting plate 120 is formed so as to be inclined at a predetermined tilting angle TA with respect to the second central axis CA2.

The front housing 130 and the rear housing 140 can be coupled to form the housing 100 of the device. The front housing 130 may include a front mounting plate 131 disposed at the upper end portion of the front housing. The front mounting plate 131 may be integrally formed with the front housing 130. The rear housing 140 may include a rear mounting plate 141 disposed at the upper end portion of the rear housing. The rear mounting plate 141 may be integrally formed with the rear housing 140. If the front housing 130 and the rear housing 140 are coupled, the front and rear mounting plates 131 and 141 may be coupled to form the mounting plate 120. The mounting plate 120 can rotatably support the head mount. The front and rear mounting plates 131 and 141 are inclined at the tilting angle TA with respect to the second central axis CA2.

The front mounting plate 131 includes a front bearing hole 132, which supports a rotation shaft of the head mount and has a semicircular shape, and a pair of front detents 133 formed in an inward end of the front mounting plate. The rear mounting plate 141 includes a rear bearing hole 142, which supports the rotation shaft of the head mount and has a semicircular shape, and a pair of rear detents 143 formed in an inward end of the rear mounting plate. When the front and rear housings 130 and 140 are assembled, the front bearing hole 132 and the rear bearing hole 142 form one bearing hole 121 (see FIG. 2) supporting the rotation shaft of the head mount, and the front detent 133 and the rear detent 143 form a detent 122 (see FIG. 2) for positioning of the head mount.

The front mounting plate 131 includes a key protrusion 134 in an outer peripheral surface of the front mounting plate, and the rear mounting plate 141 includes a key protrusion 144 in an outer peripheral surface of the rear mounting plate. The housing 100 includes a sleeve ring 150, which fixes the upper end portions of the assembled front and rear housings 130 and 140 and allows each mounting plate to sandwich the rotation shaft of the head mount. The sleeve ring 150 includes, in its inner peripheral surface, key grooves 151 to which the key protrusions 134 and 144 are fitted. The sleeve ring 150 is coupled to the upper end portions of the assembled front and rear housings 130 and 140, thereby preventing separation of the front and rear mounting plates 131 and 141 and allowing the front and rear mounting plates 131 and 141 to sandwich the rotation shaft of the head mount.

The mounting plate 120 inclined with respect to the second central axis CA2 allows the head mount to be coupled to the upper end portion of the housing in a direction tilted with respect to the second central axis CA2 and in a direction parallel with the second central axis CA2. Hereinafter, the direction tilted with respect to the second central axis is referred to as a first direction, and the direction parallel with the second central axis is referred to as a second direction. The head mount is coupled to the mounting plate 120 so as to rotate about the first central axis. The head mount rotating about the first central axis is coupled to the upper end portion of the housing in the first direction or in the second direction.

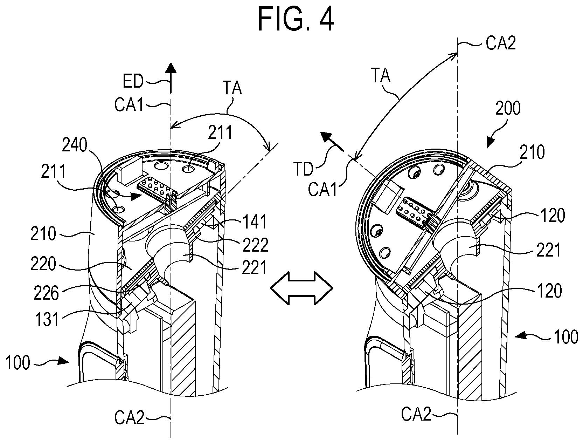

With reference to FIG. 4, the rotation of the head mount in the first direction or in the second direction is described below. FIG. 4 is a sectional view showing the head mount and the upper portion of the housing, and it further shows the respective directions of the head mount according to the rotation of the head mount.

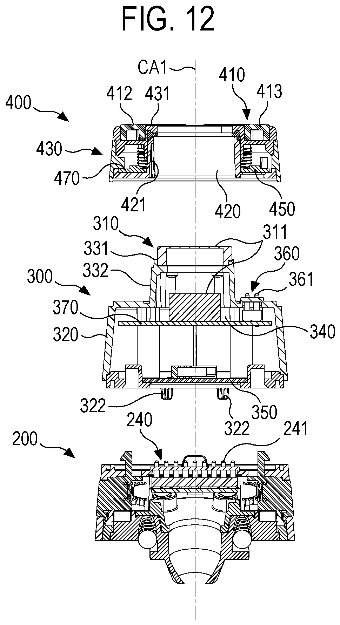

The head mount 200 includes a first cover 210 forming an outer peripheral portion and an upper portion of the head mount, as well as a second cover 220 forming a bottom portion of the head mount. The first connector 240 is disposed in an upper portion of the first cover 210. A positioning hole 211 is formed in the upper portion of the first cover 210, and a positioning pin of the first head 300 (e.g., a positioning pin 322 shown in FIG. 12) may be inserted into the positioning hole 211. The second cover 220 is inclined at the tilting angle TA with respect to the first central axis CA1 of the head mount and the second central axis CA2 of the housing. The second cover 220 includes a rotation shaft 221 protruding toward the inside of the housing 100, an engaging flange 222 protruding from the rotation shaft 221 along a circumference of the rotation shaft 221, and a coupling groove 226 formed along the circumference of the rotation shaft above the engaging flange 222. The rotation shaft 221 protrudes from the second cover 220 so as to be perpendicular to the mounting plate 120. Portions of the front and rear mounting plates 131 and 141 defining the bearing hole 121 are inserted into the coupling groove 226, and the front and rear mounting plates 131 and 141 sandwich the rotation shaft 221. Accordingly, the head mount is coupled to the mounting plate 120 of the housing so as to rotate about the first central axis CA1. When the device is used, the user can rotate the head mount 200 about the first central axis CA1. According to the rotation of the head mount 200, the head mount 200 is coupled to the mounting plate 120 in the first direction TD and in the second direction ED.

A left portion of FIG. 4 shows that the head mount 200 rotates about the first central axis CA1 to be coupled to the mounting plate 120 in the second direction ED. When the head mount 200 is coupled to the mounting plate 120 in the second direction ED, the first central axis CA1 is parallel or coaxial with the second central axis CA2. When the head mount 200 is positioned in the second direction ED, the head mount 200 and the housing 100 are positioned in an approximately straight line. When the head mount 200 is positioned in the second direction ED, the device may be in a non-use state, and the first head can be mounted to the head mount. A right portion of FIG. 4 shows that the head mount 200 rotates about the first central axis CA1 to be coupled to the mounting plate 120 in the first direction TD.

The mounting plate 120 is inclined at the tilting angle TA with respect to the second central axis CA2, and the second cover 220 is inclined at the tilting angle TA with respect to the second central axis CA2. Accordingly, as the head mount 200 rotates so as to be coupled from the second direction ED to the first direction TD, the first central axis CA1 of the head mount is gradually tilted from the second central axis CA2. When the head mount 200 is coupled to the mounting plate 120 in the first direction TD, the first central axis CA1 is inclined at the tilting angle TA with respect to the second central axis CA2.

When the head mount 200 is coupled to the mounting plate 120 in the second direction ED, the upper portion of the first cover 210 is positioned so as to be orthogonal to the second central axis CA2. When the head mount 200 is coupled to the mounting plate 120 in the first direction TD, the upper portion of the first cover 210 is tilted at the tilting angle TA with respect to the second central axis CA2. When the head mount 200 is positioned in the first direction TD, the device may be in a use state. When the head mount 200 is coupled in the first direction TD, the first central axis CA1 is tilted with respect to the second central axis CA2. Thus, the first head can be mounted to the head mount tilted with respect to the housing 100 and can be tilted with respect to the second central axis CA2. Further, the second head can be mounted to the tilted first head.

The device according to one embodiment may be configured to supply electrical power to the first head and the second head as the head mount 200 rotates about the first central axis CA1 in one direction (e.g., a counterclockwise direction of the first central axis) to be coupled to the mounting plate 120 in the first direction TD. If the user rotates the head mount 200 and positions the head mount 200 in the first direction TD, the device can be switched to a power on state where electrical power is supplied to the components of the device. If the user rotates the head mount 200 in the opposite direction opposite to the aforementioned one direction (e.g., a clockwise direction of the first central axis) and positions the head mount 200 in a direction different from the first direction TD, the device can cut off the electrical power supplied to the components and can be switched to a power off state.

The controller can control switching to the power on state and the power off state. The controller is configured to supply electrical power to the first head and the second head upon detecting that the head mount 200 is coupled to the mounting plate 120 in the first direction TD. Further, the controller is configured to supply electrical power to other electrical components provided in the housing 100 other than the first and second heads (e.g., the display 710, the speaker 720, etc.) upon detecting that the head mount 200 is coupled to the mounting plate 120 in the first direction TD. The controller connected to the electrical power source 170 can allow electrical power to be supplied from the electrical power source 170 to the first head, the second head, and other electrical components. Further, the controller is configured to cut off the electrical power supplied to the first head, the second head, and other electrical components upon detecting that coupling of the head mount 200 to the mounting plate 120 in the first direction TD is released.

As such, the controller detects the position of the head mount 200 in the first direction TD according to the rotation of the head mount, and supplies and cuts off the electrical power for the electrical components of the device. Accordingly, since the device 10 can be switched into the power on state and the power off state only by the rotation of the head mount 200, a user can easily change the power on state and the power off state of the device. Further, since the power on state and the power off state are switched only by the rotation of the head mount 200, the housing 100 of the device does not need a power button, and therefore the housing 100 may have a buttonless appearance.

According to one embodiment of the device, the housing 100 may include a sensor or switch, and the head mount 200 may include an operating portion for operating such a sensor or switch by the rotation of the head mount. The controller can detect the coupling of the head mount 200 to the mounting plate 120 in the first direction TD based on a detection signal of the sensor or the switch.

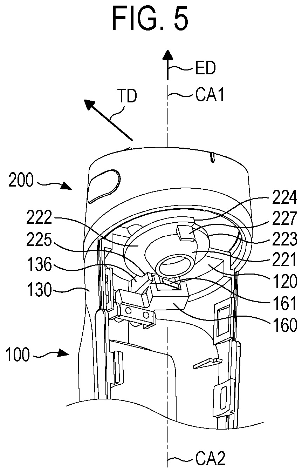

In this regard, reference is made to FIG. 5 showing the lower portion of the head mount and the upper portion of the housing. FIG. 5 is a perspective view showing the lower portion of the head mount and the upper portion of the housing.

The housing 100 includes a detection switch 160 disposed inside the housing. The detection switch 160 may be fixed to the inner peripheral surface of the front housing 130. The detection switch 160 may include a limit switch. The detection switch 160 is configured to transmit a detection signal to the controller 600 upon coupling of the head mount 200 to the mounting plate 120 in the first direction TD. As the head mount 200 rotates about the first central axis CA1, the detection switch 160 can detect the position of the head mount in the first direction and can transmit the detection signal to the controller.

The head mount 200 includes a switch operating portion 227 that can turn on or turn off the detection switch 160 by the rotation of the head mount about the first central axis CA1. The switch operating portion 227 may be formed as a pin, which is disposed in the engaging flange 222 of the rotation shaft 221 and protrudes downward.

As the head mount 200 rotates in one direction (e.g., the counterclockwise direction of the first central axis) and is coupled to the mounting plate 120 in the first direction TD, the switch operating portion 227 can press a switch terminal 161 of the detection switch 160. Accordingly, the detection switch 160 detects that the head mount 200 is coupled to the mounting plate 120 in the first direction TD, and transmits the detection signal to the controller. The controller supplies the electrical power to the respective components of the device based on the detection signal of the detection switch 160.

The engaging flange 222 of the rotation shaft has a concave edge 223, which is formed along a circumference of the engaging flange 222 and is concave toward a center of the rotation shaft 221. The concave edge 223 is formed approximately throughout a half of the engaging flange 222. Both ends of the concave edge 223 form first and second stepped portions 224 and 225, respectively. The first and second stepped portions 224 and 225 protrude with respect to the center of the rotation shaft 221. The switch operating portion 227 is located adjacent to the first stepped portion 224 and protrudes from the engaging flange 222. The front housing 130 of the housing includes a stopper 136, which protrudes from the inner peripheral surface of the front housing, and with which the first and second stepped portions 224 and 225 of the engaging flange are engaged. The stopper 136 is positioned such that the second stepped portion 225 is engaged with the stopper 136 when the head mount 200 is coupled to the mounting plate in the second direction ED. Alternatively, the stopper 136 is positioned such that the first stepped portion 224 is engaged with the stopper 136 when the head mount 200 is coupled to the mounting plate in the first direction TD.

When the head mount 200 rotates in the aforementioned one direction and is coupled to the mounting plate in the first direction TD, the switch operating portion 227 presses the switch terminal 161 of the detection switch 160, and the detection switch 160 transmits the detection signal to the controller. Further, when the head mount 200 is coupled to the mounting plate in the first direction TD, the first stepped portion 224 is engaged with the stopper 136, and the head mount 200 is prevented from rotating further. As the head mount 200 rotates in the aforementioned opposite direction, the switch terminal 161 of the detection switch 160 is released, and the detection switch 160 does not transmit the detection signal to the controller. As such, the controller can detect that the coupling of the head mount 200 in the first direction TD is released. When the head mount 200 rotates in the aforementioned opposite direction and is coupled to the mounting plate in the second direction ED, the second stepped portion 225 is engaged with the stopper 136, and the head mount 200 is prevented from rotating further.

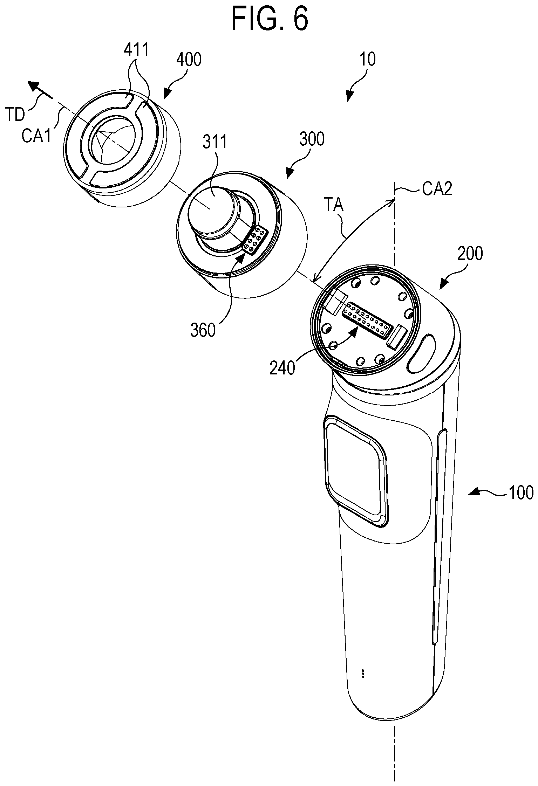

Reference is made to FIG. 6 showing a state where the head mount is coupled to the housing in the first direction. FIG. 6 is a perspective view showing that the head mount is coupled to the housing in the first direction shown in FIG. 4. The head mount 200 is coupled to the mounting plate in the first direction TD, and the first central axis CA1 is tilted at the tilting angle TA with respect to the second central axis CA2. Accordingly, the first head 300 can be mounted to the head mount 200 in a state of being tilted at the tilting angle TA, and the second head 400 can be mounted to the first head 300 along the first central axis CA1. The first head 300 and the second head 400, which are tilted with respect to the second central axis CA2 of the housing, can be applied to the target object (a user's skin) at an ergonomic angle.

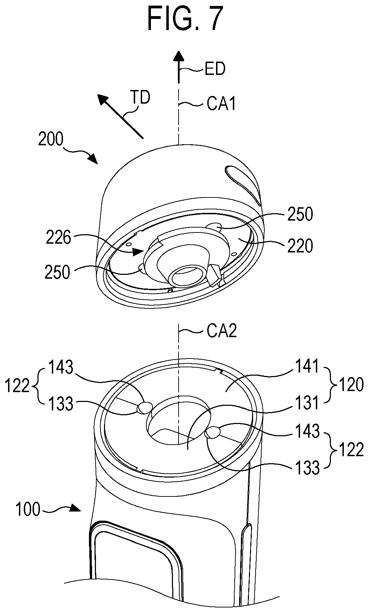

FIG. 7 is a perspective view showing the lower portion of the head mount and the upper end portion of the housing. The device according to one embodiment can fix the position of the head mount when the head mount is coupled to the mounting plate in the first direction and in the second direction. In this regard, reference is made to FIG. 7 showing the lower portion of the head mount and the upper end portion of the housing.

The head mount 200 includes balls 250 elastically pressed toward the mounting plate 120. The balls 250 is disposed so as to protrude toward the coupling groove 226 of the second cover 220, and is elastically pressed toward the mounting plate 120 by a spring. The mounting plate 120 includes a pair of detents 122 to which the ball 250 is releasably inserted. The front detent 133 of the front mounting plate and the rear detent 143 of the rear mounting plate are assembled, forming each detent 122 of the mounting plate. The pair of detents 122 are located opposite to each other with reference to the second central axis CA2. The pair of detents 122 are positioned in the mounting plate 120 such that the balls 250 are releasably inserted to the detents 122 when the head mount 200 is coupled to the mounting plate 120 in the first direction TD or the second direction ED. When a user rotates the head mount 200 about the first central axis CA1 in the aforementioned one direction and the head mount 200 is coupled to the mounting plate in the first direction TD, the balls 250 are inserted to the detents 122. Further, when a user rotates the head mount 200 about the first central axis CA1 in the aforementioned opposite direction and the head mount 200 is coupled to the mounting plate in the second direction ED, the balls 250 are inserted to the detents 122. Accordingly, due to coupling of the balls 250 and the detents 122, the head mount 200 can be fixed to the mounting plate 120 in the first direction TD or the second direction ED, and a user can feel a tactile sensation related to the rotation position of the head mount 200.

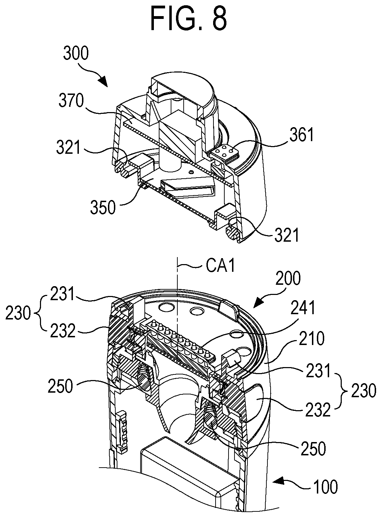

The first head is detachably mounted to the head mount. The first head can be fixedly mounted to the head mount in a state where the head mount is mounted. In this regard, reference is made to FIG. 8, which is a sectional perspective view showing the housing, the head mount, and the first head.

The head mount 200 includes a locking latch 230 for fixing the first head 300 to the head mount. The first head 300 includes a locking groove 321 to which the locking latch 230 is releasably coupled. The locking latch 230 is disposed so as to be movable toward the first central axis CA1 in the first cover 210. The locking latch 230 is elastically pressed toward the first central axis CA1 by a spring. The locking latch 230 includes an engaging hook 231, and the engaging hook 231 is releasably coupled to the locking groove 321 of the first head 300. For example, when the first head 300 is mounted to the head mount 200 along the first central axis CA1, the engaging hook 231 is moved toward the first central axis CA1 by a portion of the first head forming the locking groove 321, and can be coupled to the locking groove 321 by a force of the spring. Since the engaging hook 231 is coupled to the locking groove 321 to fix the first head 300 to the head mount 200, the first head 300 can be fixedly mounted to the head mount 200.

The locking latch 230 includes a push button 232 disposed in the outer peripheral surface of the head mount 200. The push button 232 is exposed on the first cover 210 of the head mount, and is in contact with the engaging hook 231. As a user pushes the push button 232 toward the first central axis CA1, the locking latch 230 can be moved toward the first central axis CA1. Accordingly, the engaging hook 231 can be released from the locking groove 321, and the first head 300 can be detached from the head mount 200.

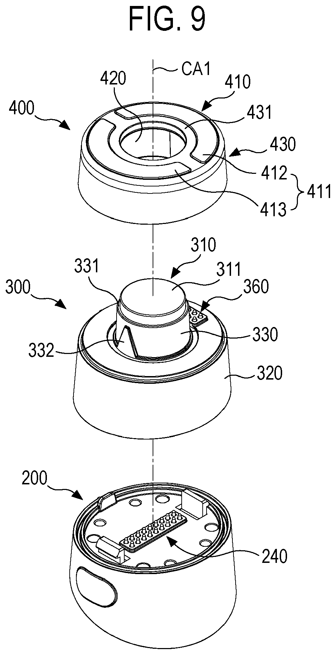

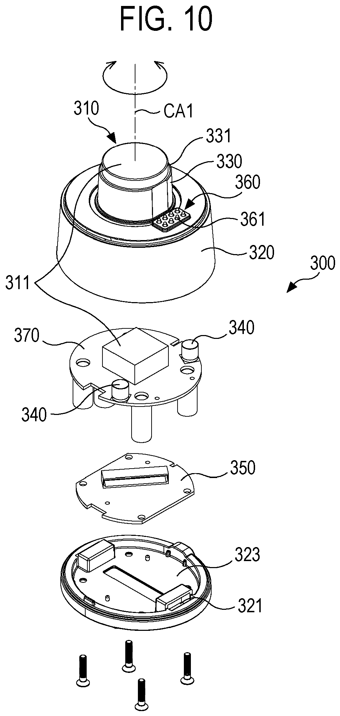

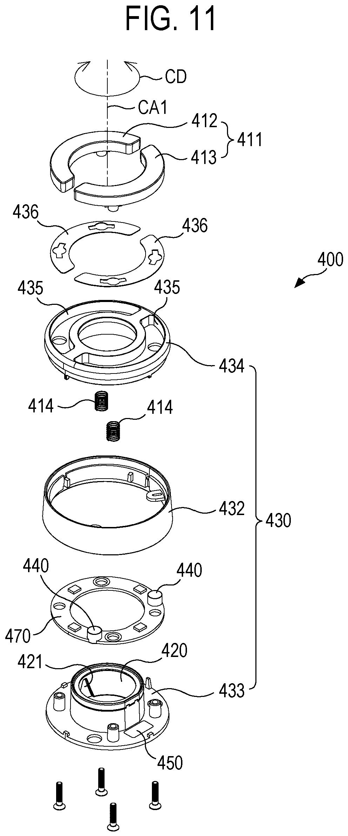

FIG. 9 is a perspective view showing the head mount, the first head, and the second head of the device according to one embodiment. FIG. 10 is an exploded perspective view of the first head shown in FIG. 9, and FIG. 11 is an exploded perspective view of the second head shown in FIG. 9. Reference is made to FIGS. 9 to 11 in the below description.

The first head 300 is detachably mounted to the head mount 200, and supports the second head 400. The second head 400 is detachably mounted to the first head 300. In a state where the second head 400 is mounted to the first head 300, the first contact portion 310 of the first head and the second contact portion 410 of the second head can make contact with the target object (e.g., the user's skin) together. According to one embodiment of the device, the first contact portion 310 is inserted to the second head 400 such that the first contact portion 310 and the second contact portion 410 can make contact with the target object together.

The first head 300 includes a disk 320 detachably mounted to the head mount 200, and a shaft 330 protruding from the disk along the first central axis CA1. The first contact portion 310 is disposed at an end of the shaft 330 spaced apart from the disk 320 along the first central axis CA1. The first contact portion 310 disposed at the end of the shaft 330 constitutes a protruding end 331 of the first head 300 formed in the shaft. The first contact portion 310 is positioned in the protruding end 331 of the first head and makes contact with the target object.

The disk 320 has a disk cover 323 forming a bottom portion of the disk. The second connector 350 may be coupled to the disk cover 323. A first head circuit board 370, which is a portion of the controller, is disposed inside the disk 320, and the second connector 350 is electrically connected to the first head circuit board 370.

The second head 400 is formed in a ring shape. The second head 400 includes a first through hole 420 into which the shaft 330 is inserted along the first central axis CA1. The first through hole 420 is perforated through the second head 400 along the first central axis CA1. The second head 400 includes a first annular body 430 seated or disposed on the disk 320 of the first head. An inner peripheral surface of the first annular body 430 forms the first through hole 420. The first annular body 430 includes an upper end 431, which is formed along a circumference of the first through hole 420. The second contact portion 410 of the second head forms the upper end 431 of the first annular body. Thus, the second contact portion 410 is formed along the circumference of the first through hole 420.

In the device according to one embodiment, the first annular body 430 of the second head includes a first annular housing 432, a first bottom cover 433 defining the first through hole 420 and coupled to the first annular housing 432, and a first top cover 434 forming the upper end of the first annular body 430. The above-described fourth connector 450 is coupled to the first bottom cover 433. A second head circuit board 470 may be disposed in an internal space which is defined by the first annular housing 432, the first bottom cover 433 and the first top cover 434. The second head circuit board 470 may be a portion of the above-described controller.

If the second head 400 is mounted to the first head 300, the shaft 330 passes through the first through hole 420, and both the first contact portion 310 and the second contact portion 410 form a portion of the device that makes contact with the target object. The shaft 330 protrudes from a center of the disk 320, and the first annular body 430 has a shape corresponding to a shape of the disk 320. Thus, in the state where the second head 400 is mounted to the first head 300, the first contact portion 310 located at the protruding end 331 of the first head and the second contact portion 410 located at the upper end of the second head 400 are positioned concentrically with respect to the first central axis CA. Specifically, in the state where the second head 400 is mounted to the first head 300, the second contact portion 410 surrounds the first contact portion 310 about the first central axis CA1. Due to the above-described arrangement of the first and second contact portions, the device can simultaneously perform the actions provided by each head to the target object.

In the state where the second head 400 is mounted to the first head 300, the second head 400 can be fixed so as not to be detached from the first head 300 along the first central axis CA1. The first head 300 includes a first magnet 340 disposed inside the disk 320, and the second head 400 includes a second magnet 440 disposed inside the first annular body 430 and positioned so as to correspond to the first magnet. In the state where the second head 400 is mounted to the first head 300, a magnetic attraction force acts by the first magnet 340 and the second magnet 440, and the magnetic attraction force can prevent the second head 400 from being detached from the first head 300 along the first central axis CA1. To prevent the second head 400 from being detached along the first central axis CA1, a mechanical fastening structure may be provided to the first head and the second head instead of the above-described magnetic attraction force.

In the state where the second head 400 is mounted to the first head 300, the second head 400 can be fixed so as not to rotate on the first head 300 about the first central axis CA1. The shaft 330 of the first head includes a key 332 in the outer peripheral surface of the shaft, and the first through hole 420 of the second head includes a key groove 421 in the inner peripheral surface of the first through hole. The key 332 and the key groove 421 are formed along the first central axis CA1, and the key 332 can be inserted to the key groove 421. Thus, the key 332 is inserted to the key groove 421, thereby preventing the rotation of the second head 400. For example, the key 332 and the key groove 421 may be formed in a shape of an isosceles triangle.

FIG. 12 is a sectional view of the head mount, the first head, and the second head shown in FIG. 9. Reference is made to FIGS. 10 to 12 in the below description.

In the device according to one embodiment, the first head 300 is configured to apply an ultrasonic wave for sonophoresis to the target object. The sonophoresis may refer to an action of an ultrasonic wave for penetrating a skin and delivering a skin care product into a skin tissue. An ultrasonic energy applied to the target object can cause cavitation of the skin tissue, increase in temperature of the skin tissue, etc., and can help the skin care product be absorbed into a lipid layer of the skin tissue.

The first contact portion 310 of the first head includes the ultrasonic element 311 for applying an ultrasonic wave for sonophoresis to the target object. The ultrasonic element 311 can generate an ultrasonic wave by means of piezoelectric effect. The ultrasonic element 311 is disposed at the protruding end 331 of the shaft, and the ultrasonic wave applying surface of the ultrasonic element 311 may form the first contact portion 310. The ultrasonic element 311 is controlled by the controller, and electrical power can be supplied from the electrical power source to the ultrasonic element. A portion of the ultrasonic element 311 may be mounted on the first head circuit board 370.

In the device according to one embodiment, the second head 400 is configured to alternately apply an electric wave to the target object. Specifically, the second contact portion 410 of the second head includes a first electrode 411 configured to alternately apply an electric wave (RF) to the target object. In the state where the second head 400 is mounted to the first head 300, the first contact portion 310 and the second contact portion 410 make contact with the target object (e.g., a user's skin) together. The first contact portion 310 applies the ultrasonic wave to the target object, and, at the same time, the second contact portion 410 can alternately apply the electric wave to the target object.

The ultrasonic wave applied to the skin can induce changes in the functional activity of proteins and increase the stretchability of collagen fibers. The electric wave alternately applied to the skin can break hydrogen bonds in a dermal layer of the skin through bulk heating and regenerate aged collagen or elastin. The application of the ultrasonic wave and the application of the electric wave are combined to promote the regeneration of aged collagen or elastin.

The first electrode 411 of the second contact portion 410 includes a pair of electrodes, and is thus formed in a bipolar type. One of the first electrodes 411 may be a plus electrode, and the other of the first electrodes 411 may be a ground electrode. The first electrode 412 of a plus electrode can emit a high frequency energy of electric wave to the target object, and the emitted high frequency energy can flow back to the first electrode 413 of a ground electrode. The second head 400 can be configured to alternately apply two electric waves having different frequencies from the first electrodes 411 to the target object.

The pair of first electrodes 412 and 413 are formed so as to be located opposite to each other with reference to the first central axis CA1. Each of the pair of first electrodes 412 and 413 is formed in a circular arc shape so as to surround a portion of the ultrasonic element 311 in the circumferential direction CD of the first central axis CA1. Each of the first electrodes 411 is formed in an approximately semicircular arc shape, and can surround an approximate half of the ultrasonic element 311. Since the pair of first electrodes 411 are disposed to surround the ultrasonic element 311, the high frequency energy of the electric wave can act to the target object within the range approximately defined by the pair of first electrodes 411.

An electrode hole 435, on which the first electrode 411 is seated, is formed in the first top cover 434. The first electrode 411 may be fixed to the first bottom cover 433 in the state of being seated on the electrode hole 435. A sealing member 436 is disposed between the first electrode 411 and the electrode hole 435. The first electrode 411 may be electrically connected to the second head circuit board 470 through a spring 414.

In the device according to one embodiment, the second contact portion 410 of the second head can apply red light to the target object. The red light can exhibit actions such as skin tissue regeneration, anti-inflammation, etc. A light emitting diode that emits the red light may be installed on the second head circuit board 470 inside the second head. The red light emitted by the red light emitting diode can be applied to the target object through the first top cover 434. Thus, the second contact portion 410 of the second head may include a light transmission portion for applying the red light to the target object. The light transmission portion may be formed as a portion of the first top cover 434, which is formed between the first electrode 411 and a circumference of the first through hole 420.

According to one embodiment of the device, the second head mounted to the first head can be replaced by an additional head that performs an action different from the action of the second head. In the device according to one embodiment, the first head is first mounted to the head mount, and the additional head is selectively mounted to the first head. The head mounted to the first head may be referred to as the second head. The additional head, which is mounted to the first head instead of the second head, may by referred to as a third head.

For example, where a user wants the actions included in the first head and the second head, the second head is mounted to the first head, and the first and the second heads can be used together. Where a user wants the actions included in the first head and the third head, the third head is mounted to the first head, and the first head and the third heads can be used together. Accordingly, the second head and the third head can detachably and selectively mounted to the first head.

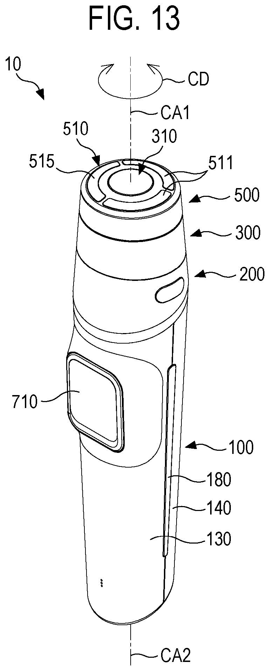

FIG. 13 is a perspective view showing the device according to one embodiment, and shows the third head mounted to the first head. Referring to FIG. 13, the device 10 according to one embodiment includes the third head 500 configured to be detachably mounted to the first head 300. The above-described controller is configured to control the third head 500. In a state where the third head 500 is mounted to the first head 300, the first head and the third head make contact with the target object (e.g., a user's skin), and can apply an ultrasonic wave and an electrical current for skin care.

The third head 500 is detachably mounted to the first head 300 along the first central axis CA1. When the device is not used, the third head 500 may be detached from the first head 300. The third head 500 includes a third contact portion 510 making contact with the target object when the device is used. The third contact portion 510 is formed in an annular shape so as to surround the first contact portion 310 of the first head in the circumferential direction CD of the first central axis CA1. Further, the third contact portion 510 is formed so as to make contact with the target object together with the first contact portion 310 when the device is used. Specifically, in the state where the third head 500 is mounted to the first head 300, the first contact portion 310 and the third contact portion 510 are positioned concentrically with respect to the first central axis CA1, and can make contact with the target object together. Therefore, when the device is used, the skin care actions included in the first head and the third head can be simultaneously performed to the target object.

The third head 500 may include an electrode that can perform the skin care action, and the third contact portion 510 may include a surface of the electrode. According to one embodiment of the device, the third contact portion 510 may be positioned at an upper end of the third head 500, and may include a second electrode 511 and a third electrode 515 that are configured to apply an electrical current to the target object.

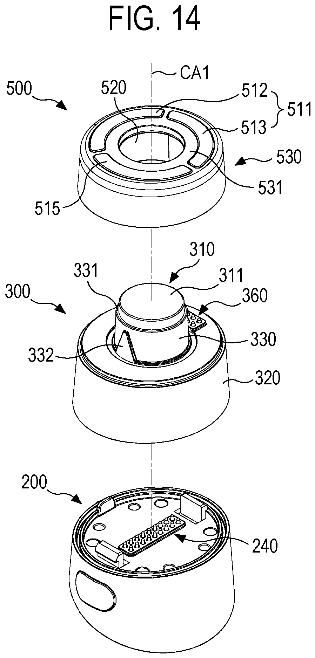

FIG. 14 is a perspective view showing the head mount, the first head and the third head shown in FIG. 13. Reference is made to FIG. 14 in the below description.

Electrical power for operation of the third head 500 may be supplied to the third head through the first head 300. For electrical connection of the first head 300 and the third head 500, the third head may include a fifth connector corresponding to the third connector 360 of the first head. The fifth connector may be disposed in a lower surface of the third head 500 so as to correspond to the third connector 360. The fifth connector may be a connector that has terminals capable of making contact with the respective pogo pins of the third connector 360 configured as a pogo pin connector. Since the third connector 360 may be configured as a pogo pin connector, coupling and decoupling of the first head 300 and the third head 500 can be easily performed. When the third head 500 is detachably mounted to the first head 300, the third connector 360 and the fifth connector are electrically connected to each other, and the third head 500 is electrically connected to the first head 300. The electrical power can be supplied from the electrical power source to the third head 500 through the head mount 200 and the first head 300 by the control of the controller.

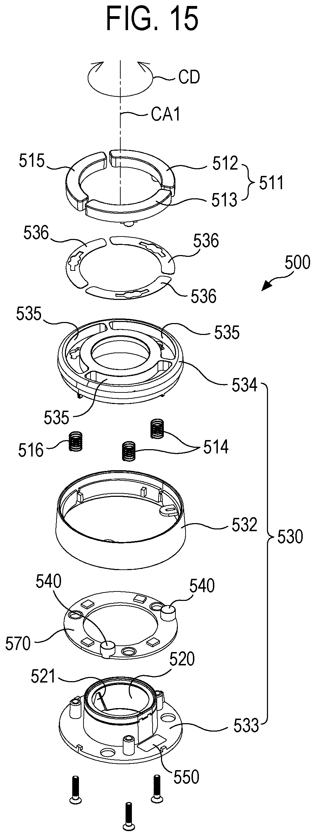

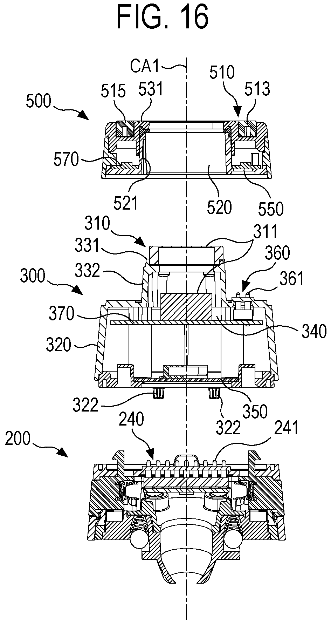

FIG. 15 is an exploded perspective view of the third head shown in FIG. 14, and FIG. 16 is a sectional view of the head mount, the first head, and the third head shown in FIG. 14. Reference is made to FIGS. 14 to 16 in the below description.

The third head 500 is detachably mounted to the first head 300, and is supported by the first head 300. The third head 500 is formed in a ring shape. According to one embodiment of the device, the first contact portion 310 is inserted to the third head 500 such that the first contact portion 310 and the third contact portion 510 can make contact with the target object together.

The third head 500 includes a second through hole 520 into which the shaft 330 of the first head is inserted along the first central axis CA1. The second through hole 520 is perforated through the third head 500 along the first central axis CA1. The third head 500 includes a second annular body 530 seated or disposed on the disk 320 of the first head. An inner peripheral surface of the second annular body 530 form the second through hole 520. The second annular body 530 includes an upper end 531, which is formed along a circumference of the second through hole 520. The third contact portion 510 of the third head forms the upper end 531 of the second annular body. Thus, the third contact portion 510 is formed along the circumference of the second through hole 520.

If the third head 500 is mounted to the first head 300, the shaft 330 passes through the second through hole 520, and both the first contact portion 310 and the third contact portion 510 form a portion of the device that makes contact with the target object. The shaft 330 protrudes from the center of the disk 320, and the second annular body 530 has a shape corresponding to the shape of the disk 320. Thus, in the state where the third head 500 is mounted to the first head 300, the first contact portion 310 located at the protruding end 331 of the first head and the third contact portion 510 located at the upper end 531 of the third head 500 are positioned concentrically with respect to the first central axis CA. Specifically, in the state where the third head 500 is mounted to the first head 300, the third contact portion 510 is configured to surround the first contact portion 310 about the first central axis CA1. Due to the above-described arrangement of the first and third contact portions, the device can simultaneously perform the actions of each head to the target object.

In the state where the third head 500 is mounted to the first head 300, the third head 500 can be fixed such that the third head is not detached from the first head 300 along the first central axis CA1. The third head 500 includes a third magnet 540 disposed inside the second annular body 530 and positioned so as to correspond to the first magnet 340 of the first head. In the state where the third head 500 is mounted to the first head 300, a magnetic attraction force acts by the first magnet 340 and the third magnet 540, and the magnetic attraction force can prevent the third head 500 from being detached from the first head 300 along the first central axis CA1.

In the state where the third head 500 is mounted to the first head 300, the third head 500 can be fixed so as not to rotate on the first head 300 about the first central axis CA1. The second through hole 520 of the third head includes, in its inner peripheral surface, a key groove 521 formed along the first central axis CA1. The key 332 of the first head is inserted to the key groove 521, thereby preventing the rotation of the third head 500.

The first contact portion 310 of the first head includes the ultrasonic element 311, thereby applying the ultrasonic wave for sonophoresis to the target object. In the device according to one embodiment, the third head 500 may be configured to apply a first electrical current for electroporation and a second electrical current for iontophoresis to the target object. Specifically, the third contact portion 510 of the third head includes a second electrode 511 configured to apply the first electrical current to the target object, and a third electrode 515 configured to apply the second electrical current to the target object. In the state where the third head 500 is mounted to the first head 300, the first contact portion 310 and the third contact portion 510 make contact with the target object (e.g., a user's skin) together. The first contact portion 310 applies the ultrasonic wave to the target object, and, at the same time, the third contact portion 510 can apply the first electrical current and the second electrical current to the target object.

The electroporation may refer to an action that applies short and intense electrostimulation to the skin to create a momentary hole in the skin barrier, and can act like an electro-needle. The iontophoresis may refer to an action of promoting the movement of ionized components contained in a skin care product, and can increase the skin permeation rate of the ionized components. The first electrical current for electroporation can create temporary holes in the skin barrier, the second electrical current for iontophoresis can inject the product into the skin, and the ultrasonic wave for sonophoresis can pull the product into the skin. The ultrasonic wave, the first electrical current, and the second electrical current may be applied to the target object in a pulsed form.

The second electrode 511 of the third contact portion includes a positive electrode 512 and a negative electrode 513 for applying the first electrical current to the target object. The positive electrode 512 and the negative electrode 513 are positioned close to each other, and apply the first electrical current for electroporation to the target object. The third contact portion includes the third electrode 515, which becomes one electrode of the two electrodes for applying the second electrical current to the target object. The third electrode 515 functions as a donor electrode, which delivers negative ions or positive ions to the skin for the iontophoresis. The third electrode 515 may be a positive electrode or a negative electrode. The above-described counter electrode disposed in the housing may have a polarity opposite to the third electrode 515. When a user holds the counter electrode of the housing with his or her hand in a state where third contact portion 510 is in contact with the skin, an electric circuit passing through the third electrode 515, a user's body, and the counter electrode is formed, and the iontophoresis can act.

In the third contact portion 510, the positive electrode 512 of the second electrode, the negative electrode 513 of the second electrode, and the third electrode 515 are spaced apart from the first central axis CA1 at an equal distance, and are arranged in the circumferential direction CD of the first central axis. Each of the positive electrode 512, the negative electrode 513, and the third electrode 515 is formed in a circular arc shape so as to surround a portion of the ultrasonic element 311 in the circumferential direction CD of the first central axis CA1. Each of the positive electrode 512, the negative electrode 513, and the third electrode 515 may be approximately formed in a circular arc shape, the subtended angle of which is less than 120 degrees.

In the device according to one embodiment, the second annular body 530 of the third head includes a second annular housing 532, a second bottom cover 533 defining the second through hole 520 and coupled to the second annular housing 532, and a second top cover 534 forming the upper end 531 of the second annular body 530. The above-described fifth connector 550 is coupled to the second bottom cover 533. Electrode holes 535, on which the second electrode 511 and the third electrode 515 are seated, are formed in the second top cover 534. The second electrode 511 and the third electrode 515 may be fixed to the second bottom cover 533 in the state of being seated on the respective electrode holes 535. Sealing members 536 are disposed between the second electrode 511 and the electrode hole 535 and between the third electrode 515 and the electrode hole 535.

A third head circuit board 570 generating the first electrical current and the second electrical current may be disposed in an internal space defined by the second annular housing 532, the second bottom cover 533, and the second top cover 534. The third head circuit board 570 may be a portion of the above-described controller. The second electrode 511 may be electrically connected to the third head circuit board 570 by a spring 514, and the third electrode 515 may be electrically connected to the third head circuit board 570 by a spring 516. The fifth connector 550 is electrically connected to the third head circuit board 570.

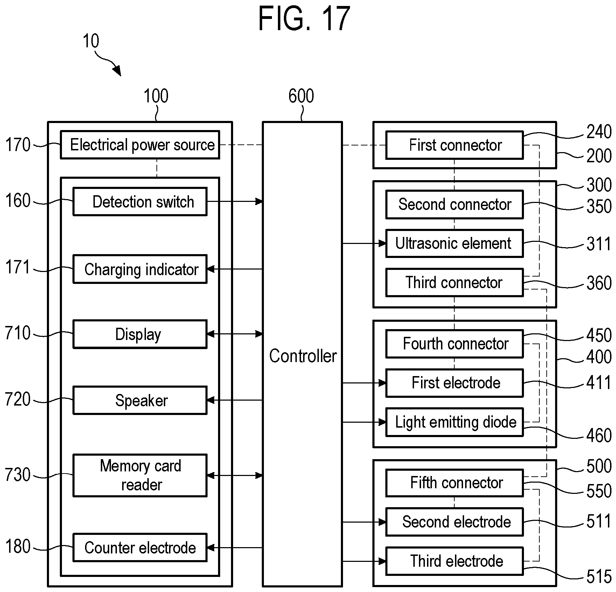

Reference is made to FIG. 17 to describe the controller of the device according to one embodiment and the operation of the device. FIG. 17 is a block diagram schematically showing the components of the device according to one embodiment. The components shown in FIG. 17 are exemplary, and the device according to other embodiment may not include a portion of the components shown in FIG. 17.

The device 10 according to one embodiment includes the controller 600 configured to control the electrical components of the device. By way of example, the controller 600 may include a micro controller unit, an ultrasonic element controlling circuit, a first electrode controlling circuit, a second electrode controlling circuit, a third electrode controlling circuit, a light emitting diode controlling circuit, a head mount detecting circuit, a display controlling circuit, a speaker controlling circuit, an electrical power source charging circuit, etc. The controller 600 may be disposed inside the housing 100. Alternatively, portions of the controller 600 may be disposed inside the first head 300 generating the ultrasonic wave for sonophoresis, inside the second head 400 generating the electric wave and the red light, and inside the third head 500 generating the first electrical current for electroporation and the second electrical current for iontophoresis.

As the head mount 200 is coupled to the housing 100 in the first direction by the rotation of the head mount, the detection switch 160 can detect the position of the head mount and can transmit the detection signal to the controller 600. The controller 600 can supply the electrical power from the electrical power source 170 to the electrical components based on the detection signal of the detection switch 160. The controller 600 can supply the electrical power from the electrical power source 170 to the ultrasonic element 311 of the first head 300, and the controller 600 can supply the electrical power from the electrical power source 170 to the display 710. Where the second head 400 is mounted to the first head 300, the controller 600 can supply the electrical power from the electrical power source 170 to the first electrode 411, and can supply the electrical power from the electrical power source 170 to the light emitting diode 460 emitting the red light. Where the third head 500 is mounted to the first head 300 instead of the second head, the controller 600 can supply the electrical power from the electrical power source 170 to the second electrode 511 and the third electrode 515 of the third head. Where the detection switch 160 does not transmit the detection signal to the controller 600 as the head mount 200 is coupled to the housing 100 in the second direction by the rotation of the head mount, the controller 600 can cut off the above-described electrical power.

The device 10 may operate in a first operation mode in the state where the first head 300 is mounted to the head mount 200 and the second head 400 is mounted to the first head. The first operation mode may be an operation mode for skin tightening. When the device 10 operates in the first operation mode, the ultrasonic wave for sonophoresis can be applied from the ultrasonic element 311 of the first contact portion 310 to the target object (e.g., a user's skin), the electric wave can be applied from the first electrode 411 of the second head 400 to the target object, and the red light can be applied to the target object through the light transmission portion of the second head 400 (the above-described first top cover).

In the first operation mode of the device 10, the controller 600 controls the ultrasonic element 311, and can allow the ultrasonic element to apply the ultrasonic wave for sonophoresis to the skin. By way of example, the ultrasonic element 311 may be configured to apply the ultrasonic wave of 1 MHz to the skin, but is not limited thereto. In the first operation mode of the device 10, the controller 600 controls the first electrode 411, and can allow the first electrode to alternately apply the electric wave to the skin. By way of example, the controller 600 is configured to allow the first electrode 411 to perform one cycle, where the first electrode 411 applies the electric wave of 1 MHz for about one second to the target object, and then the first electrode 411 applies the electric wave of 3 MHz for about one second to the target object, and thereafter the first electrode 411 stops the application of the electric wave for about one second. Further, the controller 600 may be configured to allow the first electrode 411 to perform the above-described cycle for 180 seconds.

In the first operation mode of the device 10, the ultrasonic element 311 applies the ultrasonic wave to the skin, thereby increasing the stretchability of collagen fibers by changing the functional activity of proteins. Further, the first electrode 411 alternately applies the electric wave of 1 MHz and the electric wave of 3 MHz to the skin, thereby delivering the electric wave energy to different areas under the skin surface, breaking hydrogen bonds in the skin dermis layer, and regenerating aged collagen and elastin. In the first operation mode of the device 10, since the ultrasonic wave action and the electric wave action are combined, an improved skin tightening effect can be provided to a user, and the combined actions for skin care can be provided to a user.

The device 10 may operate in a second operation mode in the state where the first head 300 is mounted to the head mount 200 and the third head 500 is mounted to the first head. The second operation mode may be an operation mode for skin nutrition. When the device 10 operates in the second operation mode, the first electrical current for electroporation is applied from the second electrode 511 of the third head 500 to the skin, the second electrical current for iontophoresis is applied from the third electrode 515 of the third head 500 or the counter electrode 180 of the housing to the skin, and the ultrasonic wave for sonophoresis is applied from the ultrasonic element 311 of the first head 300 to the skin.

In the second operation mode of the device 10, the controller 600 controls the second electrode 511 and can allow the second electrode to apply the first electrical current for electroporation to the skin. By way of example, the first electrical current applied by the second electrode 511 may have a frequency of 1 KHz under voltages of 8.6 V, 10 V, 12 V, 15 V, etc. In the second operation mode of the device 10, the controller 600 controls the third electrode 515, and can allow the third electrode to apply the second electrical current for iontophoresis to the skin. By way of example, the second electrical current applied by the third electrode 515 may have a frequency of 1 KHz under a voltage of 5 V. According to the polarities of the ionized components of the skin care product, the controller 600 can control the third electrode 515 and the counter electrode 180 such that the third electrode 515 functions as a positive electrode and the counter electrode 180 functions as a negative electrode, or such that the third electrode functions as a negative electrode and the counter electrode functions as a positive electrode.

In the second operation mode of the device 10, the action of electroporation, the action of iontophoresis, and the action of sonophoresis can be simultaneously performed to the skin. As such, since the application of the ultrasonic wave and the application of the electrical current are combined in the second operation mode, the product for skin nutrition can be absorbed to the skin more effectively, and the combined actions for skin care can be provided to a user.

The user can use the device 10 in the second operation mode by removing the second head 400 mounted to the first head 300 and mounting the third head 500 to the first head. Where the user wants to use the device 10 in the above-described first operation mode, the second head 400 can be mounted to the first head 300 instead of the third head 500. Further, since the first head 300, the second head 400, and the third head 500 are modularized components, the second head and the third head can be selectively mounted to the first head depending on the user's selection for the operation mode.

As the detection switch 160 transmits the detection signal, the display 710 can be switched into an ON state. The display 710 can display information directed to the operating state of the device 10, information directed to the above-described operation modes, and the like. The display 710 can display information for interface between the device and a user. Where the display 710 is a touch screen display, the user can input instructions related to the operation of the device 10.

By the control of the controller 600, the speaker 720 can emit information directed to the operating state of the device by sound. The speaker 720 can emit information directed to the above-described operation modes of the device 10, information directed to the operating state of the device in the above-described operation modes, and the like by sound.

The memory card reader 730 can accommodate the memory card that can include information directed to the operation and function of each head. The device 10 may include an additional head that can be mounted to the first head 300 other than the second head 400 and the third head 500. A memory card, which includes information directed to the operation and function of such an additional head, may be accommodated in the memory card reader 730, and the upgrade of the device 10 may be performed thereby.

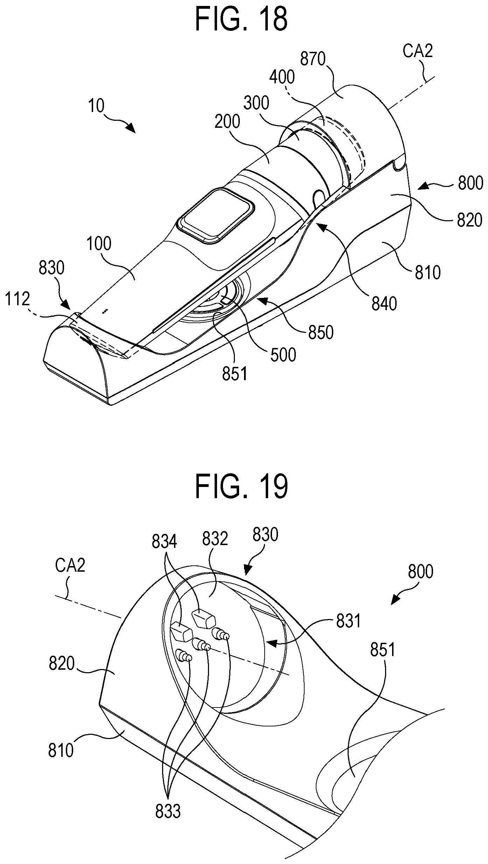

FIG. 18 is a perspective view showing a device according to one embodiment including a cradle. The device according to one embodiment may include a cradle for keeping the housing and the heads in the non-use state. The cradle may be used as a charger for charging the electrical power source disposed in the housing. The cradle may have a function of sterilizing the first and second heads or the first and third heads, which have been contacted with the user's skin. According to the embodiment, the cradle may have one or both of the function of a charger and the function of sterilization.

The device according to one embodiment can be used as an assembly in which the head mount 200, the first head 300 and the second head 400 are sequentially coupled to the housing 100 along the second central axis CA2. Further, the device according to one embodiment can be used as an assembly in which the head mount 200, the first head 300 and the third head 500 are sequentially coupled to the housing 100 along the second central axis CA2. The cradle 800 can support and keep the assembly consisting of the housing 100, the head mount 200, the first head 300 and the second head 400 (hereinafter, such an assembly is referred to as a first assembly). Further, the cradle 800 can support and keep the assembly consisting of the housing 100, the head mount 200, the first head 300 and the third head 500 (hereinafter, such an assembly is referred to as a second assembly). Further, the cradle 800 can keep the second head 400 detached from the first head or the third head 500 detached from the first head.

The cradle 800 may include a lower case 810 placed on a floor surface, and an upper case 820 coupled to the lower case 810. The cradle 800 may be used in a state where the lower case 810 of the cradle is placed on a floor surface. The cradle 800 includes docking portions 830 and 840, on which the aforementioned assemblies are placed, and to which the aforementioned assemblies are docked. The docking portions 830 and 840 may be formed as an upper portion of the upper case 820.

The cradle 800 can support the aforementioned first or second assembly along the second central axis CA2 by two docking portions. A portion of the housing, which is not supported by the cradle 800, can be situated between the two docking portions in a state where a gap is formed between a surface of the upper case 820 and the aforementioned portion of the housing. The docking portions 830 and 840 include a first docking portion 830 supporting the lower end portion 112 of the housing, and a second docking portion 840 supporting at least the head mount 200 and the first head 300. The second docking portion 840 is spaced apart from the first docking portion 830 along the second central axis CA2 of the housing. An inward end of the second docking portion 840 is located higher than an inward end of the first docking portion 830 with respect to the floor surface. Thus, the docking portions 830 and 840 are situated in a state of being tilted with respect to the floor surface, and the housing 100 can be docked to the docking portions 830 and 840 of the cradle in a state of being positioned lower than the head mount 200.

The cradle 800 includes a connection portion 850 interconnecting the first docking portion 830 and the second docking portion 840. The connection portion 850 may be a surface of the upper case 820 interconnecting the first docking portion and the second docking portion. The connection portion 850 is located lower than an imaginary line interconnecting the first docking portion and the second docking portion. Thus, in a state where the housing 100 and the head mount 200 are docked to the first docking portion and the second docking portion, respectively, a gap is formed between the connection portion 850 and the outer peripheral surface of the housing. The cradle 800 includes a head keeping recess 851 formed in the connection portion 850. The head keeping recess 851 is located between the first docking portion 830 and the second docking portion 840, and is formed so as to accommodate the second head 400 or the third head 500. FIG. 18 shows an example where the first assembly consisting of the housing, the head mount, the first head, and the second head is docked to the cradle 800 and the head keeping recess 851 accommodates the third head 500. In an example where the second assembly consisting of the housing, the head mount, the first head, and the third head is docked to the cradle 800, the head keeping recess 851 accommodates the second head 400.

A head cover 870 is rotatably disposed in the upper case 820 so as to be located adjacent to the second docking portion 840. The head cover 870 is configured to be rotatable so as to cover the first head 300 and the second head 400 docked to the second docking portion or the first head 300 and the third head 500 docked to the second docking portion.

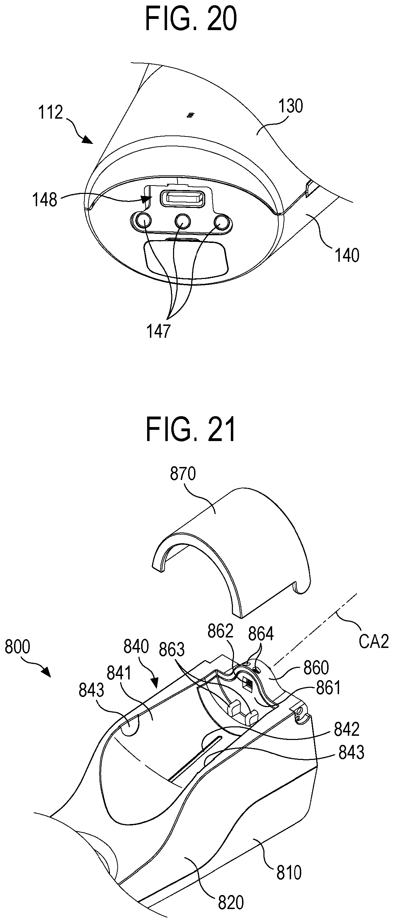

FIG. 19 is a partial perspective view of the cradle, showing the first docking portion of the cradle shown in FIG. 18. FIG. 20 is a partial perspective view showing the lower end portion of the housing shown in FIG. 18. Reference is made to FIGS. 19 and 20 in the below description.

The first docking portion 830 includes a docking hole 831. The docking hole 831 is formed along the second central axis CA2 of the housing, and surrounds the lower end portion 112 of the housing. When the lower end portion 112 of the housing is docked to the first docking portion 830, the lower end portion 112 of the housing is inserted into the docking hole 831, and the docking hole 831 receives the lower end portion of the housing along the second central axis. A docking hole cover 832 coupled to the upper case 820 may form a portion of the docking hole 831.

In the state where the aforementioned first or second assembly is docked to the cradle 800, the cradle 800 can charge the electrical power source in the housing. The cradle 800 includes a charging terminal for charging the electrical power source in the housing. The housing 100 includes a charging terminal, which is disposed in the lower end portion 112 of the housing and corresponds to the charging terminal of the cradle.

The charging terminal of the cradle is disposed in the first docking portion 830. The first docking portion 830 includes male charging terminals 833, which are disposed in a bottom of the docking hole 831 (a bottom of the docking hole cover 832) and protrude from the bottom of the docking hole. The male charging terminals 833 may be configured as a pogo pin, like the pogo pin of the above-described pogo pin connector. Each male charging terminal 833 configured as a pogo pin is configured to be pressed by the lower end portion 112 of the housing when the lower end portion 112 of the housing is docked to the first docking portion 830. As the lower end portion 112 of the housing is inserted to the docking hole 831 of the first docking portion 830 along the second central axis CA2, the male charging terminals 833 of a pogo pin can be pressed, and, at the same time, can supply a charging electrical power to the electrical power source in the housing. The first docking portion 830 has a positioning pin 834 protruding from the bottom of the docking hole 831. The positioning pin 834 can be inserted to the lower end portion 112 of the housing.

The charging terminal of the housing is disposed in the lower end portion 112 of the housing. The charging terminal of the housing includes female charging terminals 147. The female charging terminals 147 may be electrically connected to the electrical power source in the housing. By way of example, the rear housing 140 has, in its lower end, a terminal recess 148 concave upward, and the female charging terminals 147 are disposed in the terminal recess 148. The female charging terminals 147 may be formed as a terminal capable of making contact with with the male charging terminal 833 of a pogo pin, like the above-described second connector.

As the lower end portion 112 of the housing is inserted into the docking hole 831 of the first docking portion, the male charging terminals 833 are electrically connected to the female charging terminals 147, respectively. Further, the male charging terminals 833 of a pogo pin is contacted with the corresponding female charging terminal 147, and is pressed by the corresponding female charging terminal 147. As the lower end portion 112 of the housing is inserted into the docking hole 831, the positioning pin 834 is inserted to the terminal recess 148 to assist mating of the male charging terminal and the female charging terminal.

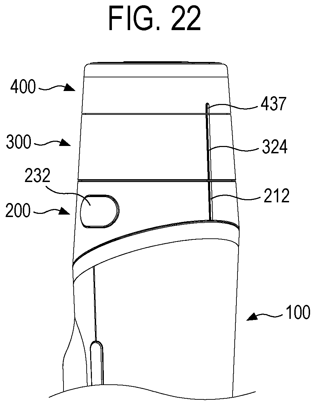

FIG. 21 is a partial perspective view of the cradle, showing the second docking portion of the cradle shown in FIG. 18. FIG. 22 is a partial perspective view showing the housing, the head mount, the first head and the second head shown in FIG. 18. Reference is made to FIGS. 21 and 22 in the below description.

The second docking portion 840 has a shape corresponding to the outer peripheral surface of the head mount 200, the outer peripheral surface of the first head 300 and the outer peripheral surface of the second head 400. The second docking portion 840 includes a docking surface 841, which is formed complementarily to the outer peripheral surface of the head mount 200, the outer peripheral surface of the first head 300 and the outer peripheral surface of the second head 400 (or the outer peripheral surface of the third head). By way of example, the docking surface 841 may be formed so as to surround a half of the head mount, a half of the first head and a half of the second head.

When the head mount and the heads are docked to the second docking portion 840, the head mount and the heads may be guided to the docking surface 841. The second docking portion 840 includes guide grooves 842 and 843, which are formed in the docking surface 841 and guide the head mount and the heads to the docking surface 841. The guide groove 842 is formed in the docking surface 841 along the second central axis CA2. Guide protrusions 212, 324 and 437 corresponding to the guide groove 842 are formed in the outer peripheral surface of the head mount 200, the outer peripheral surface of the first head 300 and the outer peripheral surface of the second head 400. A guide protrusion similar to the guide protrusion 437 of the second head is formed in the outer peripheral surface of the third head. When the head mount and the heads are docked to the second docking portion 840, the guide protrusions 212, 324, and 437 are inserted to the guide groove 842, and the head mount and the heads can be seated on the docking surface 841 without being shaken. The guide groove 843 is formed in a lateral end edge of the docking surface 841, and has a shape corresponding to a shape of the push button 232 of the head mount. When the head mount is docked to the second docking portion 840, the push button 232 can be inserted to the guide groove 843.

The cradle 800 may be configured to sterilize the heads when the cradle charges the electrical power source, or when the cradle keeps the first or second assembly. In this regard, the cradle 800 includes an ultraviolet light emitter 862, which is disposed so as to emit ultraviolet light toward the first head 300 and the second head 400 (or, toward the first head 300 and the third head).

The ultraviolet light emitter 862 is located adjacent to the second docking portion 840, and is disposed in the cradle so as to emit ultraviolet light toward the heads. The cradle includes an end wall portion 860 located adjacent to the second docking portion 840 along the second central axis CA2. The end wall portion 860 includes one end of the upper case 820 and an emitter cover 861 coupled to the one end of the upper case. In the end wall portion 860, the ultraviolet light emitter 862 is disposed in the emitter cover 861 so as to face the first head 300 and the second head 400. By way of example, the ultraviolet light emitter 862 may include an ultraviolet light emitting diode that emits ultraviolet light having a UV-C wavelength of 100 nm ~ 280 nm. The ultraviolet light emitter 862 may be disposed in the end wall portion 860 (specifically, the emitter cover 861) so as to face an approximate center of the first head 300. In the state where the head mount and the heads are docked to the second docking portion 840, the ultraviolet light emitter 862 is operated, and sterilizes the surfaces of the first head 300 and the second head 400 (or, the first head and the third head), which face the ultraviolet light emitter (e.g., the contact portions of the heads).

The end wall portion 860 may have a shape corresponding to a shape of a semicylindrical space defined by the docking surface 841. The emitter cover 861 of the end wall portion includes a stopper pin 863 protruding toward the docking surface 841. When the head mount and the heads are docked to the second docking portion 840, the stopper pin 863 can be contacted with the second head or the third head.

The cradle 800 may indicate the sterilization operation for the heads to a user. In this regard, the cradle 800 includes a sterilization indicator 864 providing information directed to the operation of the ultraviolet light emitter 862. The sterilization indicator 864 may be operated together with the operation of the ultraviolet light emitter 862 or in response to the rotation of the head cover 870. The sterilization indicator 864 is positioned in the cradle so as to be located adjacent to the second docking portion 840. Specifically, the sterilization indicator 864 is disposed in an upper end of the end wall portion 860, and can provide the information directed to the operation of the ultraviolet light emitter upward. By way of example, the sterilization indicator 864 may be a diode emitting blue light. In a state where the head cover 870 rotates to the second docking portion 840 and covers the second docking portion 840, the blue light emitted by the sterilization indicator 864 can pass through the head cover 870, and the sterilization indicator 864 can indicate the information directed to the operation of the ultraviolet light emitter to a user by the blue light.

In the device according to one embodiment, the head cover 870 is rotatably coupled to one end of the upper case 820, and is located adjacent to the second docking portion 840. A way of coupling the head cover 870 to the upper case is not limited to a rotation-permitting coupling way. The head cover 870 is formed so as to cover both the first head 300 and the second head 400 docked to the second docking portion 840 (or, the first head and the third head) and the ultraviolet light emitter 862 disposed in the end wall portion 860, and is rotatable so as to cover them. The head cover 870 is formed so as to cover both a portion of the outer peripheral surface of the first head 300 and a portion of the outer peripheral surface of the second head 400, which are not covered by the docking surface 841. If the head mount and the heads are docked to the second docking portion 840 and the head cover 870 covers the heads, the ultraviolet light emitted by the ultraviolet light emitter 862 is blocked by the head cover 870, and harmful influence on a user can be prevented thereby.

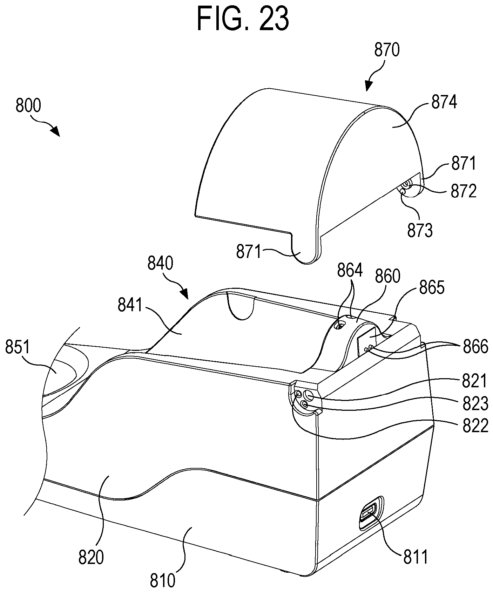

Reference is made to FIG. 23 that shows the second docking portion and the head cover of the cradle of the device according to one embodiment.

The head cover 870 partially covering the second docking portion 840 may be rotatably coupled to one end of the upper case 820. The upper case 820 includes, at its one end, a pair of hinge holes 821. The head cover 870 includes a pair of lug portions 871 protruding from a lower edge of the head cover, and each lug portion has a hinge pin 872 fitted to the hinge hole 821. The hinge pin 872 is fitted to the hinge hole 821, thereby rotatably coupling the head cover 870 to the upper case 820.

In the device according to one embodiment, the head cover 870 may be coupled to the upper case 820 so as to rotate to an open position where the heads can be inserted to the second docking potion and a closed position where the head cover covers the heads. In this regard, the upper case 820 has first and second positioning detents 822 and 823, which are located adjacent to each of the hinge holes 821 and are concave inwards. The head cover 870 has, in each lug portion 871, a positioning protrusion 873, which is located adjacent to the hinge pin 872 and is elastically coupled to the first and second positioning detents 822 and 823. When the head cover 870 rotates about the hinge pin 872, the positioning protrusion 873 is coupled to the first and second positioning detents 822 and 823. Therefore, the rotation range of the head cover 870 can be limited to the range of an angle between the first and second positioning detents 822 and 823 with respect to the hinge hole 821.

If the head cover 870 rotates to the aforementioned open position, the positioning protrusion 873 is coupled to the first positioning detent 822 and the head cover 870 can be maintained at the open position. In the state where the head cover 870 rotates to the open position, the head mount and the heads can be docked to the second docking portion 840.

If the head cover 870 rotates to the aforementioned closed position, the positioning protrusion 873 is coupled to the second positioning detent 823. Further, both lateral edges of the head cover come into contact with corresponding edges of the second docking portion 840. Therefore, a sterilization chamber for sterilizing the heads can be formed between an inner surface of the head cover 870 and the second docking portion, and the ultraviolet light emitter can emit the ultraviolet light toward the sterilization chamber.

The cradle may include a cover switch 865 detecting the rotation of the head cover 870 to the closed position. The cover switch 865 may be disposed in the end wall portion 860. As the head cover 870 rotates to the closed position, the cover switch 865 detects the rotation of the head cover, and can operate the ultraviolet light emitter and the sterilization indicator.

By way of example, the cover switch 865 includes a pogo pin 866 similar to the above-described pogo pin. When the head cover 870 rotates to the closed position, an inner surface of a closed end portion 874 of the head cover presses the cover switch 865. Therefore, the pogo pin 866 of the cover switch is pressed, the cover switch 865 detects the rotation of the head cover to the closed position, and the cover switch can generate a closing signal of the head cover.

The lower case 810 of the cradle includes a connection terminal 811 for connection to an external electrical power source. By way of example, an adapter to be connected to an external electrical power source such as a household electrical power source is connected to the connection terminal 811, and the cradle can receive electrical power from the external electrical power source. The electrical power from the external electrical power source can be supplied to the male charging terminal, the ultraviolet light emitter, and the sterilization indicator.

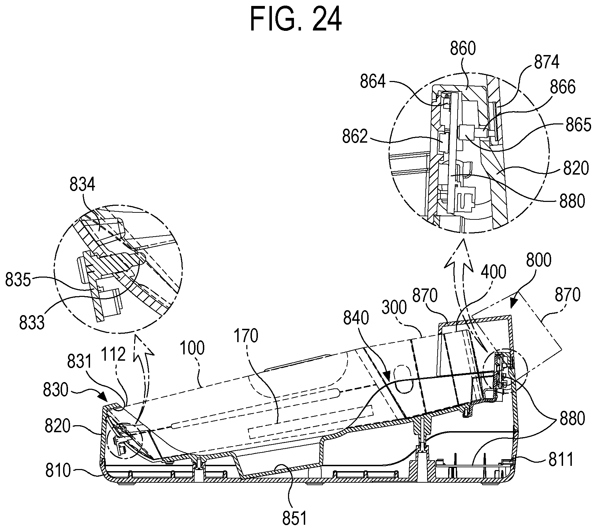

FIG. 24 is a sectional view showing a longitudinal sectional shape of the cradle shown in FIG. 18, and shows the assembly of the housing and the heads docked to the cradle by dotted lines. Reference is made to FIG. 24 in the below description.

The cradle 800 includes a cradle controller 880 controlling the operations of the components of the cradle. By way of example, the cradle controller 880 may include a board, a processing processor mounted on the board, and a plurality of circuits formed on the board, connected to the processing processor, and configured to perform respective functions of the cradle. The male charging terminal 833 is mounted on a circuit board 835, and is electrically connected to the cradle controller 880. The ultraviolet light emitter 862 is electrically connected to the cradle controller 880. The sterilization indicator 864 and the cover switch 865 are electrically connected to the cradle controller 880. The connection terminal 811 is electrically connected to the cradle controller 880, and an external electrical power source can be electrically connected to the cradle controller 880 through the connection terminal 811.

In the state where the head cover 870 rotates to the open position (the head cover shown by a dotted line in FIG. 24), the first or second assembly can be docked to the cradle 800. The lower end portion 112 of the housing is inserted into the docking hole 831 of the first docking portion, and the lower end portion 112 can be docked to the first docking portion 830. As the lower end portion 112 is docked to the first docking portion 830, the positioning pin 834 is inserted to the terminal recess of the lower end portion 112 (the terminal recess 148 shown in FIG. 20), and the female charging terminal (the female charging terminal 147 shown in FIG. 20) presses the male charging terminal 833. The electrical power source 170 in the housing 100 can be charged through the male charging terminal 833 and the female charging terminal which are electrically connected to each other.

The head cover 870 positioned to the open position rotates to the second docking portion 840 of the upper case 820, and covers the first head 300 and the second head 400. As the head cover 870 rotates to the closed position covering the first head 300 and the second head 400, the closed end portion 874 of the head cover presses the pogo pin 866 of the cover switch 865 provided in the end wall portion 860, and the cover switch 865 transmits, to the cradle controller 880, the closing signal related to the closing of the head cover. The cradle controller 880 operates the ultraviolet light emitter 862 and the sterilization indicator 864 based on the closing signal. Thus, as the head cover 870 rotates to the closed position, the sterilization action can be performed to the first head 300 and the second head 400 docked to the second docking portion 840 by the ultraviolet light emitter 862. Further, as the head cover 870 rotates to the closed position, the sterilization indicator 864 can be operated so as to provide the information directed to the operation of the ultraviolet light emitter 862. Further, the ultraviolet light emitted by the ultraviolet light emitter 862 can be blocked by the head cover 870.

By way of example, the cradle controller 880 can operate the ultraviolet light emitter 862 such that the ultraviolet light emitter 862 is operated for a predetermined time in response to the closing signal of the cover switch 865. By way of another example, the cradle controller 880 can detect the rotation of the head cover 870 to the open position, and can stop the ultraviolet light emitter 862 and the sterilization indicator 864. If the head cover 870 rotates to the open position, the pogo pin 866 of the cover switch 865 returns to the state of not being pressed, and the cover switch 865 can transmit an open signal of the head cover to the cradle controller 880. Accordingly, the cradle controller 880 can stop the operation of the ultraviolet light emitter 862 and the operation of the sterilization indicator 864.

The technical idea of the present disclosure has been described heretofore with reference to some embodiments and examples shown in the accompanying drawings. However, it is to be understood that various substitutions, modifications, and alterations that can be understood by those of ordinary skill in the technical field to which the present disclosure pertains may be made without departing from the technical idea and scope of the present disclosure. Further, it is to be understood that such substitutions, modifications, and alterations fall within the scope of the appended claims.

The disclosure extends to the following set of numbered statements:
S1. A device, comprising:
   a housing;
   a head mount configured to be coupled to the housing;
   a first head including a first contact portion making contact with a target object, wherein the first head is configured to be detachably mounted to the head mount along a first central axis of the head mount and to be electrically connected to the head mount;
   a second head including a second contact portion formed to surround the first contact portion and making contact with the target object together with the first contact portion, wherein the second head is configured to be detachably mounted to the first head along the first central axis and to be electrically connected to the first head; and
   a controller configured to control the first head and the second head.
S2. The device of statement S 1, wherein the head mount is configured to rotate about the first central axis to be coupled to an end portion of the housing in a first direction or a second direction, and
   wherein the controller is further configured to:
      supply electrical power to the first head and the second head upon detecting that
   the head mount is coupled to the end portion of the housing in the first direction; and
      cut off the electrical power upon detecting that coupling of the head mount to the end portion of the housing in the first direction is released.
S3. The device of statement S2, wherein the housing includes a detection switch disposed inside the housing and transmitting a detection signal to the controller upon coupling of the head mount to the end portion of the housing in the first direction, and
   wherein the head mount includes a switch operating portion turning on or off the detection switch by rotation of the head mount.
S4. The device of statement S2 or S3, wherein a mounting plate is formed at the end portion of the housing to which the head mount is coupled, the mounting plate being inclined at a predetermined tilting angle with respect to a second central axis of the housing,
   wherein the head mount is configured to rotate about the first central axis to be coupled to the mounting plate in the first direction or the second direction;
   wherein, when the head mount is coupled in the first direction, the first central axis of the head mount is tilted at the tilting angle with respect to the second central axis of the housing, and
   wherein, when the head mount is coupled in the second direction, the first central axis of the head mount is parallel or coaxial with the second central axis of the housing.
S5. The device of statement S4, wherein the head mount includes a ball elastically pressed toward the mounting plate, and
   wherein the mounting plate includes a detent to which the ball is releasably inserted when the head mount is coupled to the end portion of the housing in the first direction or the second direction.
S6. The device of any of the preceding statements, wherein the head mount includes a locking latch elastically pressed toward the first central axis and including a push button disposed in an outer peripheral surface of the head mount, and
   wherein the first head includes a locking groove to which the locking latch is releasably coupled.
S7. The device of any of the preceding statements, wherein the first head includes:
   a disk detachably mounted to the head mount;
   a shaft protruding from the disk along the first central axis; and
   a protruding end formed in the shaft by the first contact portion, and
   wherein the second head includes:
      a first through hole into which the shaft is inserted along the first central axis; and
      a first annular body disposed on the disk and including an upper end formed along a circumference of the first through hole by the second contact portion.
S8. The device of statement S7, wherein, in a state where the second head is mounted to the first head, the first contact portion of the protruding end and the second contact portion of the upper end are positioned concentrically with respect to the first central axis.
S9. The device of statement S7 or S8, wherein the first head includes a first magnet disposed inside the disk, and wherein the second head includes a second magnet disposed inside the first annular body and corresponding to the first magnet.
S 10. The device of statement S7, S8 or S9, wherein the shaft includes a key formed along the first central axis in an outer peripheral surface of the shaft, and wherein the first through hole includes a key groove into which the key is inserted.
S 11. The device of any of the preceding statements, further comprising:
   a third head configured to be detachably mounted to the first head along the first central axis and to be electrically connected to the first head, and including a third contact portion formed to surround the first contact portion and making contact with the target object together with the first contact portion,
   wherein the second head and the third head are detachably and selectively mounted to the first head, and
   wherein the controller is configured to control the third head.
S12. The device of statement S11, wherein the first head includes:
   a disk detachably mounted to the head mount;
   a shaft protruding from the disk along the first central axis; and
   a protruding end formed in the shaft by the first contact portion, and
   wherein the third head includes:
      a second through hole into which the shaft is inserted along the first central axis; and
      a second annular body disposed on the disk and including an upper end formed along a circumference of the second through hole by the third contact portion.
S13. The device of statement S12, wherein the first head includes a first magnet disposed inside the disk, and wherein the third head includes a third magnet disposed inside the second annular body and corresponding to the first magnet, and
   wherein the shaft includes a key formed along the first central axis in an outer peripheral surface of the shaft, and wherein the third through hole includes a key groove into which the key is inserted.
S14. The device of statement S 11, S12 or S13, wherein the first contact portion includes an ultrasonic element for applying an ultrasonic wave for sonophoresis to the target object,
   wherein one of the second contact portion and the third contact portion includes a first electrode configured to alternately apply an electric wave to the target object,
   wherein the other of the second contact portion and the third contact portion includes:
      a second electrode configured to apply a first electrical current for electroporation to the target object; and
      a third electrode configured to apply a second electrical current for iontophoresis to the target object, and
   wherein the housing includes a counter electrode, which is disposed in an outer peripheral surface of the housing and is configured to apply the second electrical current to the target object.
S15. The device of statement S 14, wherein the first electrode includes a pair of electrodes formed in a circular arc shape so as to surround a portion of the ultrasonic element in a circumferential direction of the first central axis, respectively.
S16. The device of statement S14 or S15, wherein the second electrode includes a positive electrode and a negative electrode for applying the first electrical current to the target object, and
   wherein each of the positive electrode of the second electrode, the negative electrode of the second electrode, and the third electrode is formed in a circular arc shape so as to surround a portion of the ultrasonic element in a circumferential direction of the first central axis.
S17. The device of any of statements S11 to S16, further comprising:
   an electrical power source disposed inside the housing and configured to be controlled by the controller to supply electrical power to the first head, the second head, and the third head.
S18. The device ofany of the preceding statements, further comprising:
   a display disposed on the housing, configured to display information directed to an operating state of the device, and controlled by the controller.
S19. The device of any of the preceding statements, further comprising:
   a speaker disposed inside the housing, configured to emit information directed to an operating state of the device by sound, and controlled by the controller.
S20. The device of any of the preceding statements, further comprising:
   a memory card reader disposed in the housing and being configured to accommodate a memory card, the memory card reader being connected to the controller.
S21. The device of any of the preceding statements, further comprising:
   a cradle configured to support the housing, the head mount, and the first head that are sequentially coupled along a second central axis of the housing,
   wherein the cradle includes:
      a first docking portion supporting a lower end portion of the housing; and
      a second docking portion spaced apart from the first docking portion along the second central axis and supporting the head mount and the first head.
S22. The device of statement S21, wherein the first docking portion includes a docking hole receiving the lower end portion of the housing along the second central axis, and
   wherein the second docking portion includes:
   a docking surface formed complementarily to an outer peripheral surface of the head mount and an outer peripheral surface of the first head; and
   a guide groove formed in the docking surface and guiding the head mount and the first head to the docking surface.
S23. The device of statement S21 or S22, further comprising:
   an electrical power source disposed inside the housing and configured to supply electrical power to the first head and the second head,
   wherein the housing includes a female charging terminal disposed in the lower end portion of the housing and charging the electrical power source, and
   wherein the first docking portion includes a male charging terminal electrically connected to the female charging terminal and configured to be pressed by the female charging terminal.
S24. The device of statement S21, S22 or S23, wherein the cradle includes an ultraviolet light emitter disposed so as to emit ultraviolet light toward the first head and the second head.
S25. The device of statement S24, wherein the cradle includes a sterilization indicator providing information directed to an operation of the ultraviolet light emitter.
S26. The device of statement S25, wherein the cradle includes:
   a head cover configured to be rotatable so as to cover the ultraviolet light emitter and the first and second heads docked to the second docking portion; and
   a cover switch detecting rotation of the head cover to operate the ultraviolet light emitter and the sterilization indicator.
S27. The device of any of statements S21 to S26, further comprising:
   a third head configured to be detachably mounted to the first head along the first central axis and to be electrically connected to the first head, and including a third contact portion formed to surround the first contact portion and making contact with the target object together with the first contact portion, and
   wherein the cradle includes a head keeping recess located between the first docking portion and the second docking portion and formed so as to accommodate the second head or the third head.

## Claims

1. A device, comprising:
a housing;
a head mount configured to be coupled to the housing;
a first head including a first contact portion making contact with a target object, wherein the first head is configured to be detachably mounted to the head mount along a first central axis of the head mount and to be electrically connected to the head mount;
a second head including a second contact portion formed to surround the first contact portion and making contact with the target object together with the first contact portion, wherein the second head is configured to be detachably mounted to the first head along the first central axis and to be electrically connected to the first head; and
a controller configured to control the first head and the second head.

2. The device of Claim 1, wherein the head mount is configured to rotate about the first central axis to be coupled to an end portion of the housing in a first direction or a second direction, and
wherein the controller is further configured to:
supply electrical power to the first head and the second head upon detecting that the head mount is coupled to the end portion of the housing in the first direction; and
cut off the electrical power upon detecting that coupling of the head mount to the end portion of the housing in the first direction is released,
and optionally wherein:
the housing includes a detection switch disposed inside the housing and transmitting a detection signal to the controller upon coupling of the head mount to the end portion of the housing in the first direction, and
the head mount includes a switch operating portion turning on or off the detection switch by rotation of the head mount.

3. The device of Claim 2, wherein a mounting plate is formed at the end portion of the housing to which the head mount is coupled, the mounting plate being inclined at a predetermined tilting angle with respect to a second central axis of the housing,
wherein the head mount is configured to rotate about the first central axis to be coupled to the mounting plate in the first direction or the second direction;
wherein, when the head mount is coupled in the first direction, the first central axis of the head mount is tilted at the tilting angle with respect to the second central axis of the housing, and
wherein, when the head mount is coupled in the second direction, the first central axis of the head mount is parallel or coaxial with the second central axis of the housing,
and optionally wherein:
the head mount includes a ball elastically pressed toward the mounting plate, and
the mounting plate includes a detent to which the ball is releasably inserted when the head mount is coupled to the end portion of the housing in the first direction or the second direction.

4. The device of any of the preceding claims, wherein the head mount includes a locking latch elastically pressed toward the first central axis and including a push button disposed in an outer peripheral surface of the head mount, and
wherein the first head includes a locking groove to which the locking latch is releasably coupled.

5. The device of any of the preceding claims, wherein the first head includes:
a disk detachably mounted to the head mount;
a shaft protruding from the disk along the first central axis; and
a protruding end formed in the shaft by the first contact portion, and
wherein the second head includes:
a first through hole into which the shaft is inserted along the first central axis; and
a first annular body disposed on the disk and including an upper end formed along a circumference of the first through hole by the second contact portion,
and optionally wherein:
in a state where the second head is mounted to the first head, the first contact portion of the protruding end and the second contact portion of the upper end are positioned concentrically with respect to the first central axis; and/or
the first head includes a first magnet disposed inside the disk, and wherein the second head includes a second magnet disposed inside the first annular body and corresponding to the first magnet; and/or
the shaft includes a key formed along the first central axis in an outer peripheral surface of the shaft, and wherein the first through hole includes a key groove into which the key is inserted.

6. The device of any of the preceding claims, further comprising:
a third head configured to be detachably mounted to the first head along the first central axis and to be electrically connected to the first head, and including a third contact portion formed to surround the first contact portion and making contact with the target object together with the first contact portion,
wherein the second head and the third head are detachably and selectively mounted to the first head, and
wherein the controller is configured to control the third head.

7. The device of Claim 6, wherein the first head includes:
a disk detachably mounted to the head mount;
a shaft protruding from the disk along the first central axis; and
a protruding end formed in the shaft by the first contact portion, and
wherein the third head includes:
a second through hole into which the shaft is inserted along the first central axis; and
a second annular body disposed on the disk and including an upper end formed along a circumference of the second through hole by the third contact portion,
and optionally wherein:
the first head includes a first magnet disposed inside the disk, and the third head includes a third magnet disposed inside the second annular body and corresponding to the first magnet, and
the shaft includes a key formed along the first central axis in an outer peripheral surface of the shaft, and wherein the third through hole includes a key groove into which the key is inserted.

8. The device of Claim 6 or 7, wherein the first contact portion includes an ultrasonic element for applying an ultrasonic wave for sonophoresis to the target object,
wherein one of the second contact portion and the third contact portion includes a first electrode configured to alternately apply an electric wave to the target object,
wherein the other of the second contact portion and the third contact portion includes:
a second electrode configured to apply a first electrical current for electroporation to the target object; and
a third electrode configured to apply a second electrical current for iontophoresis to the target object, and
wherein the housing includes a counter electrode, which is disposed in an outer peripheral surface of the housing and is configured to apply the second electrical current to the target object,
and optionally wherein:
the first electrode includes a pair of electrodes formed in a circular arc shape so as to surround a portion of the ultrasonic element in a circumferential direction of the first central axis, respectively; and/or
the second electrode includes a positive electrode and a negative electrode for applying the first electrical current to the target object, and wherein each of the positive electrode of the second electrode, the negative electrode of the second electrode, and the third electrode is formed in a circular arc shape so as to surround a portion of the ultrasonic element in a circumferential direction of the first central axis.

9. The device of any of claims 6 to 8, further comprising:
an electrical power source disposed inside the housing and configured to be controlled by the controller to supply electrical power to the first head, the second head, and the third head.

10. The device of any of the preceding claims, further comprising:
a display disposed on the housing, configured to display information directed to an operating state of the device, and controlled by the controller; and/or
a speaker disposed inside the housing, configured to emit information directed to an operating state of the device by sound, and controlled by the controller; and/or
a memory card reader disposed in the housing and being configured to accommodate a memory card, the memory card reader being connected to the controller.

11. The device of any of the preceding claims, further comprising:
a cradle configured to support the housing, the head mount, and the first head that are sequentially coupled along a second central axis of the housing,
wherein the cradle includes:
a first docking portion supporting a lower end portion of the housing; and
a second docking portion spaced apart from the first docking portion along the second central axis and supporting the head mount and the first head.

12. The device of Claim 11, wherein the first docking portion includes a docking hole receiving the lower end portion of the housing along the second central axis, and
wherein the second docking portion includes:
a docking surface formed complementarily to an outer peripheral surface of the head mount and an outer peripheral surface of the first head; and
a guide groove formed in the docking surface and guiding the head mount and the first head to the docking surface.

13. The device of Claim 11 or 12, further comprising:
an electrical power source disposed inside the housing and configured to supply electrical power to the first head and the second head,
wherein the housing includes a female charging terminal disposed in the lower end portion of the housing and charging the electrical power source, and
wherein the first docking portion includes a male charging terminal electrically connected to the female charging terminal and configured to be pressed by the female charging terminal.

14. The device of Claim 11, 12 or 13, wherein the cradle includes an ultraviolet light emitter disposed so as to emit ultraviolet light toward the first head and the second head,
optionally wherein the cradle includes a sterilization indicator providing information directed to an operation of the ultraviolet light emitter,
and further optionally wherein the cradle includes:
a head cover configured to be rotatable so as to cover the ultraviolet light emitter and the first and second heads docked to the second docking portion; and
a cover switch detecting rotation of the head cover to operate the ultraviolet light emitter and the sterilization indicator.

15. The device of any of claims 11 to 14, further comprising:
a third head configured to be detachably mounted to the first head along the first central axis and to be electrically connected to the first head, and including a third contact portion formed to surround the first contact portion and making contact with the target object together with the first contact portion, and
wherein the cradle includes a head keeping recess located between the first docking portion and the second docking portion and formed so as to accommodate the second head or the third head.
